# FASCICULE DE BREVET EUROPEEN

(11) **EP 2 941 413 B1**
(45) Date de publication et mention de la délivrance du brevet: **19.10.2016**
(21) Numéro de dépôt: 13703130.8
(22) Date de dépôt: 07.01.2013
(51) Int. Cl.: C07C 253/30, C07F 15/00, C07C 6/04, C07C 57/02, C07C 69/593, C07C 255/30

(54) **PROCÉDÉ DE SYNTHÈSE DE NITRILE-ACIDE/ESTER OMEGA INSATURÉ DANS LEQUEL ON ALTERNE DE MANIÈRE CONSÉCUTIVE DEUX TYPES DE MÉTATHÈSE CROISÉE PROCÉDÉ SWING**
VERFAHREN ZUR HERSTELLUNG VON OMEGA-UNGESÄTTIGTEN NITRILSÄUREN/ESTERN MIT ZWEI ARTEN VON ALTERNIERENDEN KREUZMETATHESEN UND SCHWINGVERFAHREN DAFÜR
PROCESS FOR THE SYNTHESIS OF OMEGA-UNSATURATED NITRILE-ACID/ESTER IN WHICH TWO TYPES OF CROSS METATHESIS ARE ALTERNATED CONSECUTIVELY, SWING PROCESS

(43) Date de publication de la demande: 11.11.2015
(73) Titulaire: Arkema France, 92700 Colombes (FR)
(72) Inventeur: DUBOIS, Jean-Luc, F-69390 Millery (FR); COUTURIER, Jean-Luc, F-69006 Lyon (FR)
(74) Mandataire: Hérard, Elise
(86) Numéro de dépôt international: PCT/IB2013/000017
(87) Numéro de publication internationale: WO 2014/106766

(56) Documents cités:
- WO-A1-2011/138051

## Description

### DOMAINE DE L'INVENTION

L'invention concerne un procédé amélioré de synthèse de nitrile-acide ou nitrile-ester oméga-insaturé par métathèse à partir d'acides ou esters gras insaturés.

Les composés nitrile-acide ou nitrile-ester oméga-insaturés sont utilisés notamment pour la fabrication d'acides ou d'esters α,ω-amino-alcanoïques à longue chaîne, c'est-à-dire dont la chaîne comporte au moins 8 atomes de carbones, eux-mêmes utilisés dans l'industrie des polymères de spécialité, notamment des polyamides de performance.

### ARRIERE-PLAN TECHNIQUE

L'industrie des polyamides utilise toute une gamme de monomères formés à partir de diamines et de diacides, de lactames, et surtout à partir d' ω-aminoacides. Ces derniers sont définis par la longueur de chaîne méthylène (CH₂)ₙ séparant deux fonctions amide -CO-NH-. Ces monomères sont généralement fabriqués par voie de synthèse chimique en utilisant comme matières premières des oléfines en C2 à C4, des cycloalcanes ou du benzène, hydrocarbures issus de sources fossiles. Par exemple, les oléfines en C2 servent à fabriquer l'aminoacide en C9 utilisé dans le Pelargon russe ; les oléfines en C4 servent à fabriquer l'héxaméthylène diamine ; le laurolactame et le caprolactame sont fabriqués à partir de cycloalcanes ; l'acide adipique, le Nylon 6 et le Nylon 6,6 sont fabriqués à partir de benzène.

L'évolution actuelle en matière d'environnement conduit dans les domaines de l'énergie et de la chimie à privilégier l'exploitation des matières premières naturelles provenant d'une source renouvelable. C'est la raison pour laquelle certains travaux ont été repris pour élaborer sur le plan industriel des procédés utilisant des acides/esters gras comme matière première de fabrication de ces monomères.

Ce type d'approche n'a que peu d'exemples industriels. Un des rares exemples de procédé industriel utilisant un acide gras naturel comme matière première est celui de la fabrication à partir de l'acide ricinoléique extrait de l'huile de ricin, de l'acide amino-11-undécanoïque, qui est à la base de la synthèse du Rilsan^{®} 11. Ce procédé est décrit dans l'ouvrage « Les Procédés de Pétrochimie » de A. Chauvel et al. paru aux Editions TECHNIP (1986). L'acide amino-11-undécanoïque est obtenu en plusieurs étapes. La première consiste en une méthanolyse de l'huile de ricin en milieu basique produisant le ricinoléate de méthyle qui est ensuite soumis à une pyrolyse pour obtenir d'une part l'heptanaldéhyde et, d'autre part l'undécylénate de méthyle. Ce dernier est passé en forme acide par hydrolyse. L'acide formé est ensuite soumis à une hydrobromuration pour donner l'acide ω-bromé d'où l'on passe par ammoniation à l'acide amino-11-undécanoïque.

Dans cette voie « bio », les principaux travaux ont porté sur la synthèse de l'acide amino-9-nonanoïque qui est le précurseur du Nylon 9 à partir de l'acide oléique d'origine naturelle.

La demanderesse a pour sa part conduit des travaux dans ce domaine. Elle a décrit dans le document de brevet FR2912741 un procédé de synthèse de toute une gamme de ce type d'aminoacide/ester à partir d'un acide/ester gras naturel à longue chaîne en soumettant ce dernier à une réaction de métathèse catalytique croisée avec un composé insaturé comportant une fonction nitrile suivie d'une hydrogénation. Dans le document de brevet FR2938533, elle a également décrit un procédé de synthèse d'acides ω-amino-alcanoïques ou de leurs esters à partir d'acides gras naturels insaturés à longue chaîne transitant par un composé intermédiaire de type nitrile ω-insaturé dont l'une des variantes met en oeuvre dans la phase finale une métathèse croisée du nitrile ω-insaturé avec un composé de type acrylate. Enfin, dans le document de brevet FR2941694, elle a décrit une variante du procédé précédent dans laquelle le composé intermédiaire est du type dinitrile insaturé. Ces procédés, conduisent à l'issue d'une étape d'hydrogénation de la fonction nitrile et de la double liaison à la fabrication d'aminoacides. Dans le document de brevet FR2959742, et la demande internationale WO 2011/138051, le procédé décrit a pour objet d'améliorer les performances des procédés mettant en oeuvre successivement une métathèse croisée et une hydrogénation.

Dans ces procédés de l'art antérieur, les réactions de métathèse croisée, généralement effectuées entre un nitrile gras oméga insaturé et un acrylate, ou entre un ester gras omega insaturé et l'acrylonitrile, conduisent non seulement au produit désiré qui est un nitrile-ester, mais aussi à des produits issus de réaction d'homométathèse des corps gras tels que respectivement des dinitriles et des diesters. En augmentant les quantités de catalyseur mises en oeuvre, les temps de réaction, et/ou les ratios entre les réactifs, il est possible de convertir ces co-produits issus de l'homométathèse en nitrile-ester, cependant ces solutions s'avèrent couteuses et peu productives.

D'autre part, les produits des réactions d'homométathèse sont des produits lourds à longue chaîne, diesters ou dinitriles, qu'il est difficile de séparer du milieu réactionnel et de purifier. Les applications de ce type de produit sont limitées, et bien souvent déconnectées des applications industrielles recherchées pour les nitriles esters.

Il existe donc un réel besoin de mettre au point un procédé de synthèse de nitrile-ester/acide dans lequel la quantité de co-produits issus des réactions d'homométathèse est réduite.

La Demanderesse a trouvé que, de manière surprenante, il était possible de minimiser les quantités de co-produits, diesters ou dinitriles gras, issus des réactions d'homométathèse, en opérant différentes réactions de métathèses croisées consécutives et alternées.

### DESCRIPTION DETAILLEE DE L'INVENTION

Dans la présente description, on précise que lorsqu'il est fait référence à des intervalles, les expressions du type « allant de...à » ou « comportant/comprenant de... à » incluent les bornes de l'intervalle. A l'inverse, les expressions du type « compris entre...et... » excluent les bornes de l'intervalle.

Sauf mention contraire, les pourcentages exprimés sont des pourcentages molaires. Sauf mention contraire, les paramètres auxquels il est fait référence sont mesurés à pression atmosphérique.

La présente invention a donc pour objet un procédé de synthèse d'un nitrile-acide ou nitrile-ester à chaîne longue à rendement amélioré par métathèse croisée.

Par métathèse croisée au sens de l'invention on entend le schéma réactionnel général suivant : 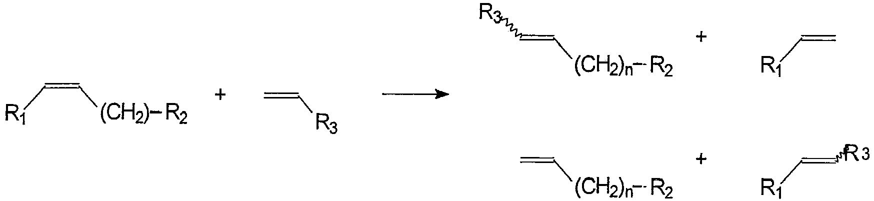
R₁=H, un radical alkyle en C1 à C11 comportant le cas échéant une fonction hydroxyle, ou (CH₂)ₘ-R₄,
R₂=COOR₅ ou CN,
R₄=H, ou R₂ dans le cas où l'on part d'un diester ou dinitrile à chaîne longue,
R₅= H, un radical alkyle de 1 à 11 atomes de carbone, ou encore le reste d'un diol, du glycérol, d'un diglycéride ou d'un triglycéride ;
R₃=COOR₆, CN, H dans le cas de l'éthylène, CH₃ dans le cas du prop-1-ène, ou CH₂-CH₃ dans le cas du but-1-ène.
R₆=radical alkyle de 1 à 4 atomes de carbone,
m est un indice entier compris dans la gamme 0 à 11,
n est un indice entier compris dans la gamme 2 à 13,
R2 différent de R3.

Le procédé selon l'invention est caractérisé en ce qu'il comprend une étape dans laquelle on alterne de manière consécutive deux types de métathèse croisée sur un composé **I** insaturé comportant au moins une fonction trivalente, nitrile, acide ou ester.

Un premier type de métathèse croisée (ci-après « **mc1** ») est effectué avec un composé acrylique **II** choisi parmi l'acrylonitrile, l'acide acrylique ou un ester acrylique. Dans ce type de métathèse croisée mc1, l'un des composés **I** ou **II** comporte une fonction nitrile et l'autre de ces composés comporte une fonction acide ou ester.

De préférence, la réaction de métathèse considérée dans ce premier type mc1 est une réaction de métathèse croisée entre un composé acide ou ester mono-insaturé généralement issu de l'oléochimie avec l'acrylonitrile ou une réaction de métathèse croisée entre un composé nitrile insaturé généralement issu de l'oléochimie avec un composé acrylique, acide ou acrylate, et dans ce cas de préférence l'acrylate de méthyle.

Le procédé a été développé en vue de l'exploitation de matières premières issues de sources naturelles renouvelables. Il peut cependant aussi bien s'appliquer aux composés insaturés analogues obtenus par synthèse chimique.

Ce type de métathèse mc1 est de préférence réalisé en présence d'un catalyseur de métathèse de type carbène de ruthénium comme décrit plus loin.

Un deuxième type de métathèse croisée (ci-après « **mc2** ») est effectué avec un composé alcène léger **III** en C2 à C4, de préférence avec l'éthylène. Dans la présente déscription de l'invention, on utilise indifféremment les termes synonymes « oléfine » ou « alcène » pour désigner un hydrocarbure non fonctionnalisé comprenant de 2 à 4 atomes de carbone et une seule double liaison covalente entre deux des atomes de carbone.

Par « alternance » de ces deux types de métathèse dans le procédé de l'invention, on entend la succession, au moins une fois, d'une métathèse croisée de type 1 (mc1) puis d'une métathèse croisée de type 2 (mc2), suivant le cycle mc1-mc2 ; ou inversement la succession, au moins une fois, d'une métathèse de type 2 (mc2) puis d'une métathèse de type 1 (mc1), soit le cycle mc2-mc1 ; étant entendu que le type de métathèse effectué en premier sur le composé I de départ peut être aussi bien mc1 que mc2.

A titre d'exemple, le procédé selon l'invention comprend un des cycles suivants : mc1-mc2, mc2-mc1, mc1-mc2-mc1, mc2-mc1-mc2, mc1-mc2-mc1-mc2, mc2-mc1-mc2-mc1, mc1-mc2-mc1-mc2-mc1, mc2-mc1-mc2-mc1-mc2, etc.

L'alternance de ces deux types de métathèse mc1 et mc2 peut être réalisée autant de fois qu'on le souhaite, ou autant de fois que nécessaire, selon l'amélioration de rendement visée en nitrile-acide(ester).

On peut citer comme exemple particulier le cycle [mc1 avec acrylonitrile] - [mc2 avec éthylène « éthénolyse »] - [mc1 avec acrylonitrile].

Cette alternance de métathèses croisées selon l'invention est également appelée ci-après « cycle SWING » ou « procédé SWING ».

Par « de manière consécutive », on entend la succession des deux types de métathèse définis ci-dessus, sans étape de séparation intermédiaire autre que la séparation éventuelle de produits légers de nombre de carbone inférieur à 15, et de préférence inférieur à 5, comme des oléfines légères par exemple ou des composés acryliques résiduels (acrylonitrile ou acrylate de méthyle par exemple).

Par exemple dans le cycle mc2-mc1-mc2, la deuxième métathèse (de type 1) consécutive à une première métathèse (de type 2) est réalisée directement sur les produits obtenus à l'issue de ladite première métathèse, et une troisième métathèse (de type 2) consécutive à la deuxième métathèse est réalisée directement sur les produits obtenus à l'issue de cette deuxième métathèse.

C'est cette alternance de métathèses consécutives de type 1 et 2 selon le procédé de l'invention, qui permet d'obtenir un rendement amélioré en nitrile-ester(acide) oméga-mono-insaturé, comparé au rendement obtenu avec une seule métathèse de type mc1 ou une seule métathèse de type mc2.

Comme composé I de départ utilisable dans le procédé de l'invention, on entend tout particulièrement un acide ou ester gras insaturé, de préférence d'origine naturelle, de formule :

R1-CH=CH-[(CH₂)_{q}-CH=CH]ₚ -(CH₂)ₙ-R2

dans laquelle :
R1 est H, un radical alkyle de 1 à 11 atomes de carbone comportant le cas échéant une fonction hydroxyle, ou (CH₂)ₘ-R4
m est un indice entier compris dans la gamme de 0 à 11,
n est un indice entier compris dans la gamme de 2 à 13,
p est un indice entier, p étant égal à 0, 1 ou 2,
q est un indice égal à 0 ou 1,
R2 est COOR5 ou CN,
R4 est H ou R2
R5 est choisi parmi : H, un radical alkyle de 1 à 11 atomes de carbone ou un radical comportant deux ou trois atomes de carbone porteur de une ou deux fonction(s) hydroxyle(s), ou encore le reste d'un di-glycéride ou d'un tri-glycéride dans lequel chaque acide gras dudit reste de glycéride est indifféremment saturé ou insaturé.

Les doubles liaisons C=C du composé I peuvent être en conformation cis ou trans.

Le cycle SWING peut aussi bien être mis en oeuvre sur des corps gras omega insaturés dont la double liaison se trouve en fin de chaîne, que sur des corps gras dont la double liaison est interne, mais on utilise de préférence les corps gras omega insaturés.

Selon un mode de réalisation avantageux du procédé de l'invention, on utilise un composé I de départ dans lequel l'indice p est égal à 0 et R1 est égal à H, c'est-à-dire un composé mono-insaturé et oméga-insaturé de formule CH₂=CH-(CH₂)ₙ-R2.

De préférence dans ce cas, le cycle de métathèses commence par une métathèse de type mc1 sur le composé I, ceci pour accéder à des composés bi-fonctionnels.

On forme ainsi un nitrile-ester, et un coproduit diester ou dinitrile à longue chaîne.

La métathèse croisée suivante est une réaction impliquant un alcène (mc2) conduisant à une consommation du diester ou dinitrile à longue chaine, converti alors en partie en composé oméga-insaturé. Un nouveau cycle de type mc1 permet de convertir les composés oméga-insaturés en nitrile-ester.

De préférence, le composé I insaturé est un acide ou ester gras mono-insaturé oméga-insaturé de formule CH₂=CH-(CH₂)ₙ-COOR5.

De préférence, R5 est H ou un radical alkyle comprenant de 1 à 11 atomes de carbone, de préférence de 2 à 11 atomes de carbone.

Selon un premier mode de réalisation avantageux du procédé de l'invention, des esters (acides) gras oméga insaturés, y compris lorsque ces esters (acides) gras omega insaturés sont présents sous la forme de triglycérides, sont mis en oeuvre avec de l'acrylonitrile, au cours d'une première étape de métathèse croisée, en présence d'un premier catalyseur de métathèse, en ajoutant de manière continue le catalyseur. Pendant cette première étape, où l'acrylonitrile réagit, de l'éthylène se dégage de la réaction, et un mélange de nitrile-ester (nitrile-acide) et de diester (diacide) est formé. A l'issue de cette première étape, une seconde étape de métathèse croisée est engagée, en utilisant cette fois l'éthylène comme réactif, et un second catalyseur de métathèse. A l'issue de cette seconde étape de métathèse croisée, la proportion de diester (diacide) issu de la réaction d'homométathèse de la première étape, est significativement réduite, car il a été converti en ester (acide) gras omega insaturé - réactif initial de la première étape.

Dans une troisième étape de métathèse croisée, le mélange produit issu de la seconde étape, est à nouveau mis en oeuvre en présence d'acrylonitrile et d'un troisième catalyseur de métathèse. Le mélange obtenu à l'issue de la troisième étape de métathèse croisée contient une plus forte proportion de nitrile-ester (nitrile-acide) que le mélange issu uniquement de la réaction de métathèse croisée impliquant l'acrylonitrile, de même durée de contact avec l'acrylonitrile et engageant les mêmes quantités de catalyseur.

Selon un autre mode de réalisation avantageux du procédé de l'invention, le composé I insaturé est un nitrile gras mono-insaturé oméga-insaturé de formule CH₂=CH-(CH₂)ₙ-CN.

A titre d'exemple, des nitriles gras oméga insaturés sont mis en oeuvre dans la procédé de l'invention avec des acrylates, tels que l'acrylate de méthyle, au cours d'une première étape de métathèse croisée, en présence d'un premier catalyseur de métathèse ajouté de préférence de manière continue. Pendant cette première étape, où l'acrylate réagit, de l'éthylène se dégage de la réaction, et un mélange de nitrile-ester et de dinitrile se forme. A l'issue de cette première étape, une seconde étape de métathèse croisée est engagée, en utilisant cette fois l'éthylène comme réactif, et un second catalyseur de métathèse. A l'issue de cette seconde étape de métathèse croisée, la proportion de dinitrile issu de la réaction d'homométathèse de la première étape, est significativement réduite, car il a été converti en nitrile gras omega insaturé - réactif initial de la première étape. Dans une troisième étape de métathèse croisée, le mélange produit issu de la seconde étape, est à nouveau mis en oeuvre en présence d'acrylate et d'un troisième catalyseur de métathèse. Le mélange obtenu à l'issue de la troisième étape de métathèse croisée, contient une plus forte proportion de nitrile-ester que le mélange issu uniquement de la réaction de métathèse croisée impliquant l'acrylate de méthyle, de même durée de contact avec l'acrylate de méthyle et engageant les mêmes quantités de catalyseur.

Selon encore un autre mode de réalisation avantageux du procédé de l'invention, on utilise comme produit de départ un composé I dans lequel l'indice p est différent de 0 et/ou R1 est différent de H. Dans ce cas, le cycle de métathèses commence de préférence par une métathèse de type mc2, ceci pour accéder à des oléfines plus courtes et en particulier des composés oméga insaturés. Les composés oméga insaturés sont préférés pour les métathèses de type mc1.

D'autre part, la métathèse de type mc2 initiale conduit à des oléfines qui sont plus facilement séparées du milieu réactionnel constitué alors de nitriles ou esters gras du fait de leurs différences de propriétés physico-chimiques. En commençant par une métathèse de type mc2, il est donc facile de conserver en mélange les composés lourds n'ayant pas réagit, les produits de la réaction de métathèse croisée (esters ou nitriles omega insaturés), et les produits issus des réactions d'homométathèse.

A titre d'exemple, on met en oeuvre des acides gras insaturés sous la forme de triglycérides ou d'esters, avec un alcène léger en C2 à C4 (éthylène, propylène, but-1-ène, but-2-ène) dans une première étape de métathèse croisée de type mc2 (appelée éthénolyse dans le cas de l'éthylène), effectuée en présence d'un premier catalyseur de métathèse, ajouté de préférence de manière continue.

Pendant cette première étape, où l'alcène réagit, des alcènes se dégagent de la réaction, et des triglycérides contenant des acides insaturés plus courts, y compris omega-insaturés se forment. A l'issue de cette première étape, une seconde étape de métathèse croisée est engagée, en utilisant cette fois de l'acrylonitrile comme réactif (métathèse de type mc1), et un second catalyseur de métathèse. A l'issue de cette seconde étape de métathèse croisée, la proportion d'ester insaturé issu de la réaction de la première étape, est significativement réduite, car il a été converti en nitrile-ester gras insaturé. Avantageusement, le triglycéride obtenu est ensuite hydrolysé ou alcoolysé pour libérer le glycérol et récupérer un nitrile-acide ou un nitrile-ester gras.

Ce mode de réalisation à partir de triglycérides est par exemple réalisé comme suit :

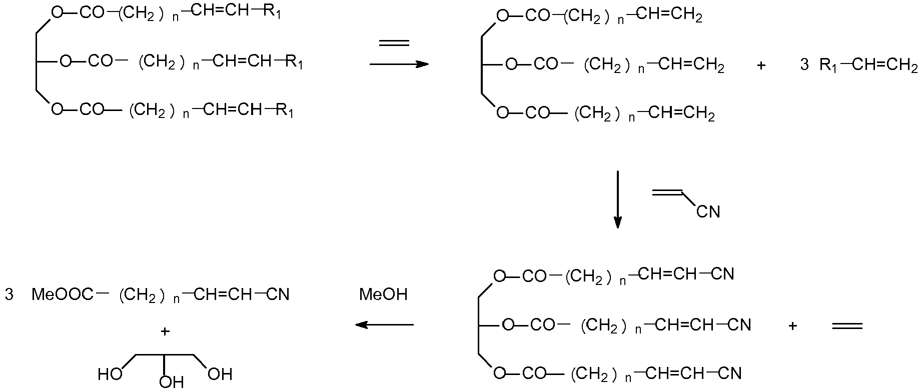

Un deuxième exemple de ce mode de réalisation mc2-mc1 est schématisé ci-dessous : 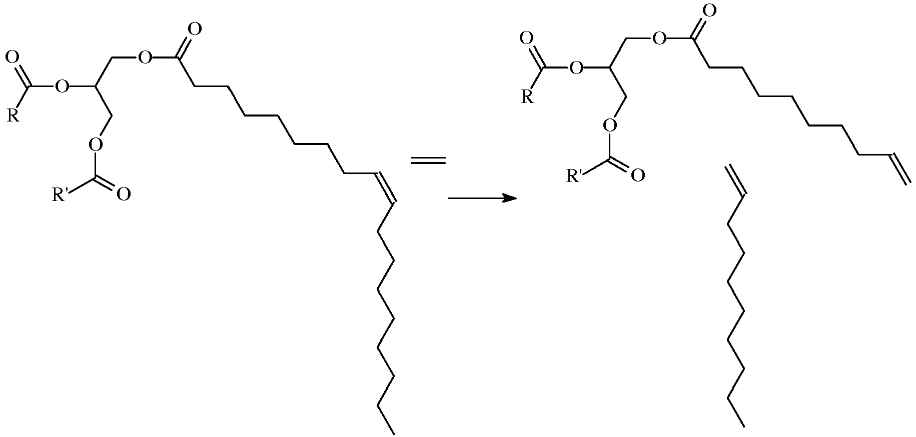 avec dégagement de 1-décène par distillation :

On conserve dans le milieu tous les triglycérides, en mélange avec les produits d'homométathèse entre triglycérides.

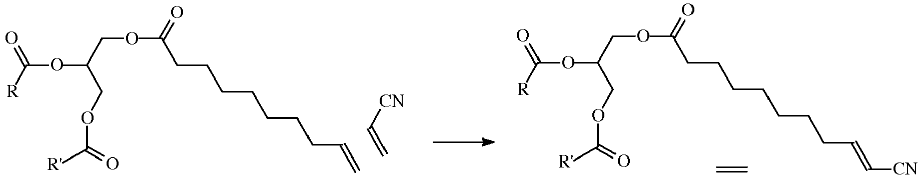

Une nouvelle métathèse croisée mc2 permet de poursuivre la conversion des triglycérides et de convertir les produits d'homométathèse de triglycérides.

Les catalyseurs de métathèse mis en oeuvre dans les étapes successives mc1 et mc2 peuvent être identiques ou différents. De préférence, on utilise des catalyseurs différents entre les étapes de métathèse croisées impliquant de l'éthylène et celles impliquant des acrylates ou de l'acrylonitrile.

La pression et la température de réaction de ces deux types de métathèses peuvent être identiques ou différentes. De préférence, la température de réaction est plus faible lors de mc2, notamment lors de la réaction d'éthénolyse, et la pression est plus élevée pour faciliter la dissolution de l'alcène (notamment l'éthylène), que dans le cas de métathèse croisée mc1 impliquant des réactifs de type acrylique.

La réaction de métathèse croisée mc1 avec un composé de type acrylique est réalisée dans des conditions parfaitement connues. La réaction de métathèse est réalisée de préférence à une température de réaction comprise entre 20 et 120 °C et la pression est comprise entre 1 et 30 bars en présence d'un catalyseur à base de ruthénium. Elle sera de préférence conduite à basse pression comprise entre 1 et 10 bars et de façon plus préférée à la pression atmosphérique lorsque la métathèse croisée conduit à la formation d'un composé léger par exemple de l'éthylène pour en permettre un dégagement aisé. La réaction peut être conduite sans solvant ou en présence de solvant, comme par exemple le toluène, les xylènes, le dichlorométhane, le benzene, le chlorobenzene, le dimethylcarbonate, le diethylcarbonate, ou leurs mélanges.

La réaction de métathèse croisée mc2, par exemple avec l'éthylène appelée éthénolyse, est réalisée de préférence à une température de réaction comprise entre 20 et 120 °C et la pression est comprise entre 1 et 30 bars en présence d'un catalyseur à base de ruthénium. Elle sera de préférence conduite sous pression comprise entre 1 et 20 bars et de façon plus préférée à une pression supérieure à 5 bars pour permettre une dissolution aisée de l'éthylène dans la phase organique. La réaction est conduite dans le milieu réactionnel, c'est-à-dire avec les solvants et résidus de catalyseurs, de l'étape de métathèse croisée antérieure. De préférence, une étape de purge pour éliminer le composé acrylique résiduel précède l'étape d'éthénolyse. De même, une étape de purge pour éliminer les alcènes longs résiduels après une métathèse croisée de type mc2 sur un ester ayant une instauration interne est de préférence effectuée.

La séparation des produits de la réaction se trouve grandement facilitée du fait de la plus faible quantité de produits d'homométathèse présents dans les produits de la réaction. En fin de cycle swing, le milieu est constitué principalement de l'ester(ou acide) nitrile, de l'ester(ou acide) ou du nitrile initial, des produits d'homométathèse. Les produits obtenus peuvent être séparés par différentes techniques du fait de leurs différences importantes de propriétés physicochimiques : distillation à pression atmosphérique ou sous pression réduite (écart important des points d'ébullition et des tensions de vapeur), cristallisation (écart important des points de fusion), extraction liquide-liquide (écart important des solubilités des produits du fait de la différence apportée par le nombre de groupements fonctionnels). De préférence, la technique de séparation retenue sera la distillation, car tous les produits sont distillables sous pression réduite.

L'avantage principal du procédé swing de l'invention est qu'il conduit directement :
- à des nitrile-acides ou des nitrile-esters insaturés avec des sélectivités supérieures à 75%, de préférence supérieures à 80%, voire supérieures à 90% ;
- les produits d'homométathèse représentant moins de 25%, voire moins de 10% par rapport à la quantité totale de produits du procédé swing.

L'invention a également pour objet un procédé de synthèse d'un α,ω-aminoester (ou acide) saturé, de préférence à longue chaîne, c'est-à-dire comportant de 6 à 17 atomes de carbone, à partir d'un composé I insaturé comportant au moins une fonction trivalente, nitrile, acide ou ester, caractérisé en ce qu'il comprend,
- une première étape de synthèse d'un nitrile-ester(acide) mono-insaturé par métathèses croisées selon le cycle swing de l'invention, dans lequel on alterne de manière consécutive deux types de métathèses croisées mc1 et mc2, tels que définis plus haut, en commençant par l'un ou l'autre de ces types de métathèse ; puis
- une étape d'hydrogénation du nitrile-ester(acide) mono-insaturé obtenu à l'étape précédente.

Les α,ω-aminoesters (acides) issus de procédé de l'invention sont fabriqués à partir de nitriles-esters(acides) exempts de co-produits d'homométathèse. Ils permettent ainsi la fabrication de polymères de meilleure qualité, avec des coûts de production réduits.

La formule du nitrile-ester (acide) et donc celle de l'α,ω-aminoester (acide) synthétisé dépendent essentiellement de celle du composé I de départ.

Dans le cas d'un composé I de départ issu de l'oléochimie, c'est-à-dire obtenu à partir d'esters ou acides gras naturels renouvelables, R₁ est soit H, soit un radical alkyle, soit un radical alkyle fonctionnel comportant une fonction trivalente (CN, COOH ou COOR).

R₁ est H lorsque l'ester gras naturel est par exemple soumis à une éthénolyse ou dans certains cas à une pyrolyse. La formule de l'α,ω-aminoester/acide obtenu est alors directement liée au radical -(CH₂)ₙ- de l'ester gras. C'est ainsi que n est égal à 7 avec l'acide oléique, à 4 avec l'acide pétrosélénique, à 8 à partir d'acide (ester) ricinoléique soumis à une pyrolyse, à 10 à partir d'acide (ester) lesquérolique soumis à une pyrolyse etc..., comme décrit dans le document de brevet FR2912741.

R₁ est un radical alkyle lorsque dans (CH₂)ₘ-R₄, R₄ est H. Ceci correspond à l'utilisation dans le procédé d'un acide gras naturel mono-insaturé, tel que par exemple les acides oléique, palmitoléique, pétrosélénique, lauroléique etc...

R₁ est un radical alkyle fonctionnel lorsque dans (CH₂)ₘ-R₄, R₄ est un radical représentant une fonction trivalente CN, COOH ou COOR qui est identique à R₂. Le composé est alors sous la forme diacide, diester ou dinitrile. La formule du composé présente alors une symétrie. L'obtention de ce type de composés, par métathèse notamment, est décrite dans les documents de brevet FR2912741, FR2938533, et FR2941694 citées précédemment.

En ce qui concerne le composé acrylique de mc1, le choix de la fonction trivalente R₃ est lié à la nature de la fonction trivalente de l'autre composé, R₃ devant être nitrile lorsque R₂ est ester/acide et réciproquement ester/acide lorsque R₂ est nitrile.

Avantageusement, lorsqu'on procède à partir d'une huile, la première métathèse croisée de type mc2 conduit toujours à un triglycéride (composé lourd) facilement séparé des oléfines légères qui se forment. On procède alors à une métathèse de type mc1 avec de l'acrylonitrile, ce qui donne des composés de type triglycérides portant des fonctions nitriles. Après une étape d'hydrolyse ou de transestérification à basse température on obtient du glycérol, des esters ou acides gras à chaine longue et des nitriles acides (ou esters) à chaine courte, qu'il devient alors possible de séparer. Par rapport à un procédé dans lequel on aurait isolé des esters ou acides oméga insaturés avant de procéder à la métathèse croisée de type mc1, on gagne en nombre d'étapes de séparation et donc en consommation d'énergie.

Avantageusement, le procédé de l'invention comprend une métathèse croisée mc1 avec l'acrylonitrile réalisée sur un composé I choisi parmi l'acide 9-décénoïque ou le 9-décénoate de méthyle, issus de l'éthénolyse (ou autre métathèse croisée avec un alcène) de l'acide oléique ou de l'oléate de méthyle ou encore d'un triglycéride, de l'acide 10-undécénoïque ou le 10-undécénoate de méthyle, issus du craquage de l'acide ricinoléique ou du ricinoléate de méthyle, l'acide oléique ou l'oléate de méthyle, l'acide 9-octadécènedioïque ou le 9-octadécènedioate de méthyle, issus de l'homométathèse ou de la fermentation de l'acide oléique, l'acide érucique et l'érucate de méthyle, l'acide 12-tridécénoïque ou le 12-tridécénoate de méthyle issus de l'acide lesquérolique.

Avantageusement, le procédé de l'invention comprend une métathèse croisée mc1 avec l'ester(acide) acrylique réalisée sur un composé I choisi parmi le 9-décènenitrile issu de l'acide 9-décénoïque, le 10-undécènenitrile issu de l'acide 10-undécénoïque, le 9-octadécènenitrile ou oléonitrile, issu de l'acide oléique, le 9-octadécènedinitrile, issu de l'acide 9-octadécènedioïque, l'éruconitrile, le 12-tridécénonitrile issu de l'acide lesquérolique.

La catalyse de la réaction de métathèse a fait l'objet de très nombreux travaux et le développement de systèmes catalytiques sophistiqués. On peut citer par exemple les complexes au tungstène développés par Schrock et al (J. Am. Chem. Soc. 108 (1986) 2771 ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Letters 1 (1999) 953) qui sont des complexes ruthénium-benzylidène opérant en catalyse homogène. Enfin, des travaux ont été conduits pour la réalisation de catalyseurs immobilisés, c'est-à-dire de catalyseurs dont le principe actif est celui du catalyseur homogène, notamment les complexes ruthénium-carbène, immobilisés sur un support inactif. L'objectif de ces travaux est d'augmenter la sélectivité de la réaction de métathèse croisée vis-à-vis des réactions parasites telles que les « homo-métathèses » entre les réactifs mis en présence. Ils portent non seulement sur la structure des catalyseurs mais également sur l'incidence du milieu réactionnel et les additifs pouvant être introduits.
Le procédé selon l'invention utilise avantageusement un catalyseur de métathèse de type ruthénium-carbène.

Les catalyseurs ruthénium-carbène sont choisis de préférence parmi les catalyseurs chargés ou non-chargés de formule générale :

(X1)ₐ (X2)_{b}Ru(carbène C) (L1)_{c}(L2)_{d} (L3)ₑ

dans laquelle :
- a, b, c, d et e sont des nombres entiers, identiques ou différents, avec a et b égaux à 0, 1 ou 2 ; c,d et e égaux à 0, 1, 2, 3 ou 4;
- X1 et X2, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, un alkyle, le tétraphénylborate et dérivés. X1 ou X2 peuvent être liés à (L1 ou L2) ou au (carbène C) de façon à former un ligand bidenté ou chélate sur le ruthénium ; et
- L1, L2 et L3 identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéthers, ou un carbène hétérocyclique ; L1, L2 ou L3 peuvent être liés au (carbène C) de façon à former un ligand bidenté ou chélate, ou tridenté.
Le (carbène C) est représenté par la formule générale : C_(R1)_(R2) pour laquelle R1 et R2 sont des groupes identiques ou différents tels que l'hydrogène ou tout autre groupe hydrocarboné, fonctionnalisé ou non, de type saturé, insaturé, cyclique, aromatique, branché et/ou linéaire. A titre d'exemples, on pourra citer les complexes du ruthénium alkylidènes, benzylidène, benzylidène éther, ou cumulènes tels que les vinylidènes Ru=C=CHR ou allénylidènes Ru=C=C=CR1 R2 ou indénylidènes.

Un groupe fonctionnel (permettant d'améliorer la rétention du complexe du ruthénium dans un liquide ionique) peut être greffé sur au moins l'un des ligands X1, X2, L1, L2, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

Le catalyseur de métathèse peut être éventuellement hétérogénéisé sur un support afin de faciliter sa récupération/recyclage.

Les catalyseurs de métathèse croisée du procédé de l'invention sont de préférence des carbènes du ruthénium décrits par exemple dans Aldrichimica Acta, vol 40, N°2, 2007, p.45-52.

Des exemples de tels catalyseurs sont les catalyseurs de Grubbs, les catalyseurs Hoveyda-Grubbs, les catalyseurs Piers-Grubbs, et autres catalyseurs de métathèse du même type, qu'ils soient dits de « 1^{ère} génération » ou de « 2^{ème} génération ».

Les catalyseurs de Grubbs sont basés sur un atome de ruthénium entouré par 5 ligands :
- 2 ligands anioniques, tels que des halogénures
- 2 ligands donneurs d'électrons, tels que les tri-alkyl-phosphines, ou les carbènes N-hétérocycliques saturés (appelés « ligand NHC »),
- un groupement alkylidène, tels que des groupements méthylènes « =CR₂ » substitués ou non.
On classe ces catalyseurs de métathèse en deux catégories, suivant la nature de leurs ligands L donneurs d'électrons :
- ceux qui contiennent 2 ligands phosphine (et pas de ligand NHC saturé), développés en premier, sont des catalyseurs de type 1^{ère} génération.
- ceux qui contiennent 1 ligand NHC saturé (carbène hétérocyclique), sont des catalyseurs de type 2^{ème} génération.
Un type de catalyseur dit « Hoveyda-Grubbs », contient parmi les ligands donneurs d'électrons, un ligand chélatant benzylidène-éther, et soit une phosphine (1^{ère} génération) soit un ligand NHC saturé (2^{ème} génération) généralement substitué par deux mésityls (Mes).
Un autre type de catalyseur dit « Piers-Grubbs », forme un complexe cationique à 4 ligands qui ne nécessite pas la dissociation d'un ligand avant la réaction.

Les catalyseurs Grubbs de 1^{ère} génération s'avèrent particulièrement bien adaptés pour la métathèse mc2 selon le procédé de l'invention. Ceux de 2^{ème} génération s'avèrent plutôt mieux adaptés pour la métathèse mc1. Les catalyseurs Hoveyda-Grubbs de 2^{ème} génération sont particulièrement bien adaptés aux métathèses mc1 avec l'acrylonitrile.

De préférence, pour les réactions de métathèse croisée mc2 avec une oléfine légère, L1, L2, L3 ne sont pas des carbènes hétérocycliques. Les catalyseurs de première génération sont préférés dans le cas des métathèses de type mc2.

De préférence, pour les réactions de métathèse croisée mc1 impliquant un composé acrylique (acide acrylique, acrylonitrile, acrylate d'alkyle), L1, L2 ou L3 est un carbène hétérocyclique, de préférence N hétérocyclique saturé. Les catalyseurs de seconde génération sont préférés dans le cas des métathèses de type mc1.

Des catalyseurs préférés pour les étapes de métathèse croisées mc1 impliquant un composé acrylique sont par exemple le catalyseur Umicore M51 (commercialisé par la société Umicore) de formule (A) ci-dessous, et le catalyseur Hoveyda-Grubbs de 2^{nde} génération dénommé aussi Hoveyda II (commercialisé par Sigma-Aldrich, et Materia) de formule (B) ci-dessous.

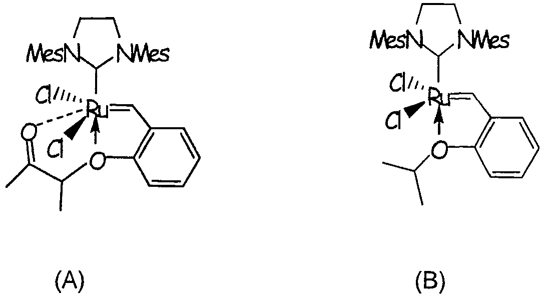

D'autres composés commercialisés par la société Oméga-cat sont également bien adaptés (groupes C et D ci-dessous), ou bien ceux commercialisés par Aldrich (E à J ci-après), ou encore ceux commercialisés par STREM et Umicore (M2, M3₁, M4₁ et M5₁ ci-après).

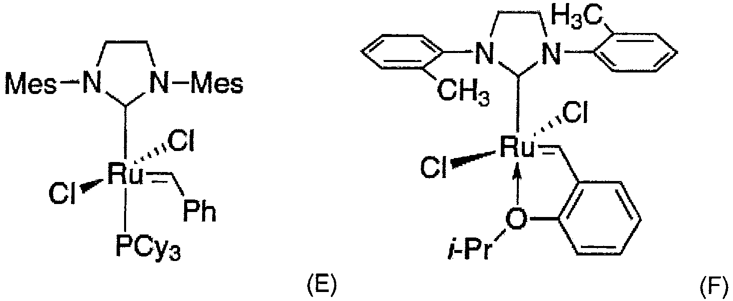

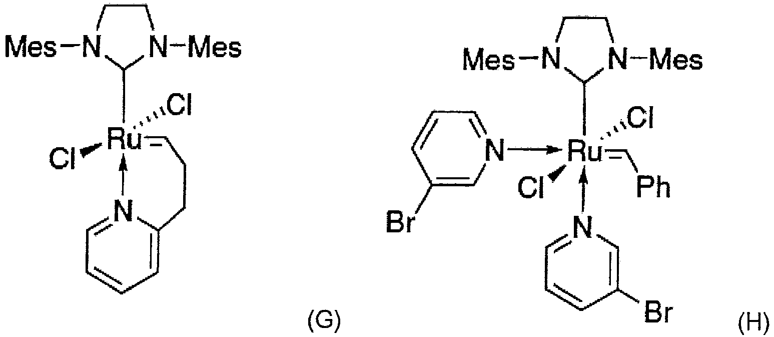

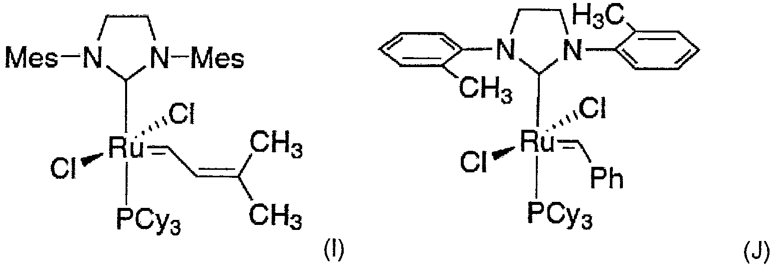

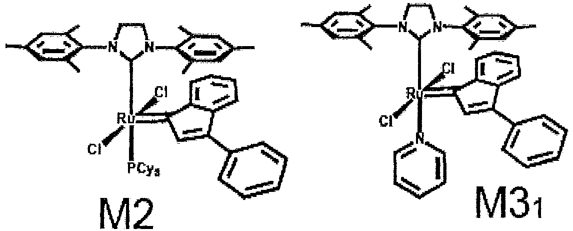

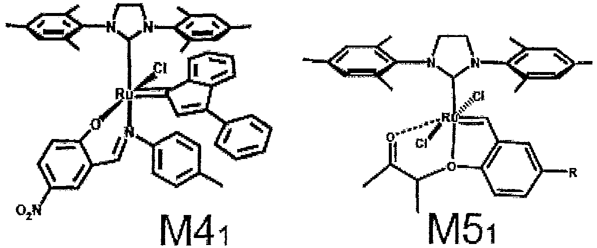

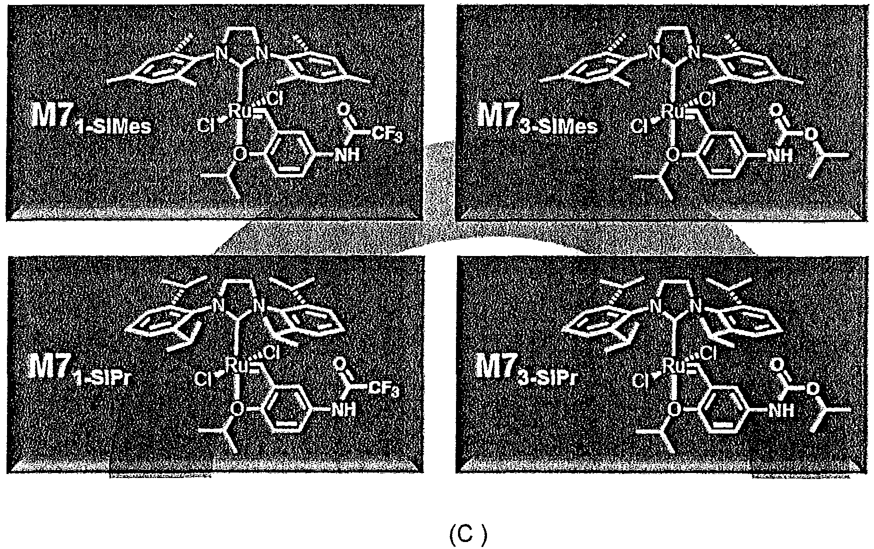

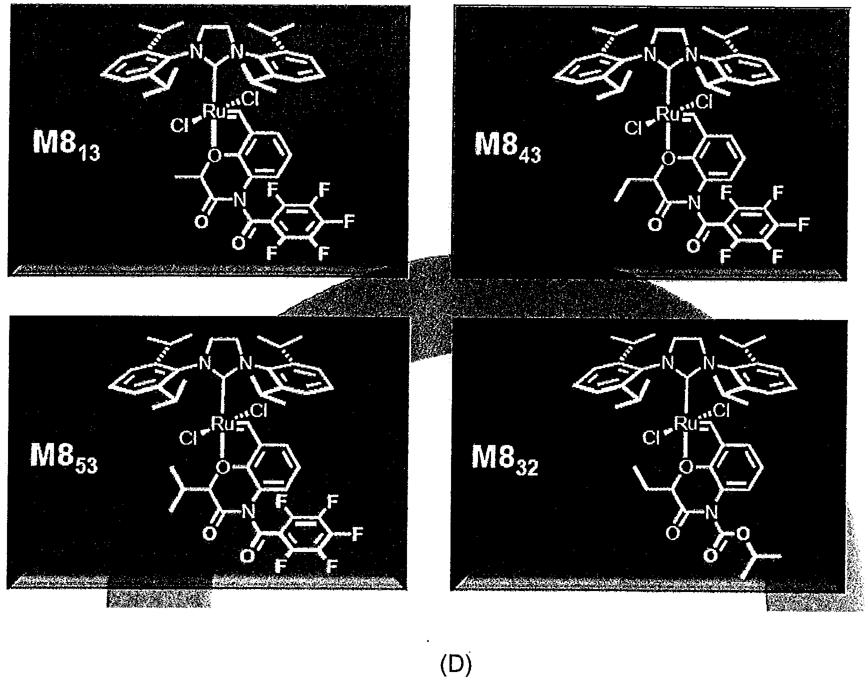

Les catalyseurs de métathèse croisée mc2 avec des oléfines légères, notamment les catalyseurs d'éthénolyse, du procédé de l'invention sont de préférence des carbènes du ruthénium décrits par exemple dans Aldrichimica Acta, vol 40, N°2, 2007, p.45-52, et dans Chemfiles Vol9, N°6. Des catalyseurs préférés pour les étapes de métathèse croisée mc2 sont notamment le catalyseur M1 (commercialisé par la société Umicore), et les catalyseurs Grubbs de 1ère génération (commercialisés par Sigma-Aldrich, et Materia) de formule (N, O et P) ci-dessous.

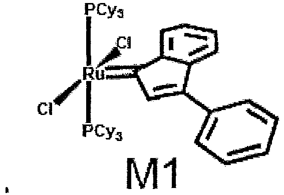

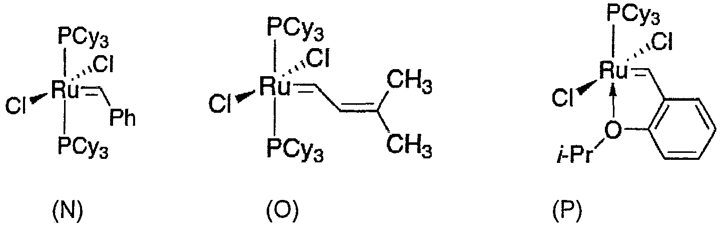

La quantité de catalyseur de métathèse au ruthénium introduite lors du cycle SWING est choisie de telle sorte qu'il assure toute la transformation possible du réactif (composé I) contenu dans la charge. On observe que ledit (ou lesdits) catalyseur(s), dans les conditions opérationnelles de l'étape de métathèse, est (sont) au terme de la réaction transformé ; il s'épuise ou se désactive et perd son activité catalytique en terme de métathèse - il sera désigné par la suite par le qualificatif de « dégradé » pour ladite réaction. En procédé batch, la quantité de catalyseur peut aisément être réglée pour donner la conversion souhaitée à dégradation totale du catalyseur. Avantageusement, la quantité de catalyseur présente pendant la réaction de métathèse est caractérisée par un « turnover number » ou « nombre de rotations », égal au rapport molaire [réactif qui a réagi] / [catalyseur], compris dans la gamme de 5000 à 200000, et de préférence de 10 000 à 50 000.

Le temps de réaction de métathèse est choisi en fonction des réactifs et des conditions opératoires mis en oeuvre et pour atteindre le terme de la réaction.

La métathèse étant une réaction équilibrée, il est préférable de déplacer cet équilibre pour aller vers la conversion totale. Pour ce faire, dans le cas où le co-produit de la réaction est une oléfine légère telle que l'éthylène, il est aisé de « dégazer » le réacteur de temps en temps ou en continu pour forcer l'élimination des légers et donc aller à conversion totale. Dans le cas où le co-produit est une oléfine plus lourde, éventuellement fonctionnelle, l'opération d'extraction est plus délicate dans la mesure où il faut maintenir dans le milieu réactionnel, les deux réactifs et le catalyseur. Par ailleurs, si on doit séparer au moins en partie le nitrile-ester(acide) insaturé par distillation et éliminer les légers avant une éventuelle hydrogénation, il est préférable d'opérer de sorte que le catalyseur de métathèse reste dans la fraction lourde avec le nitrile-ester(acide) pour l'utiliser dans sa fonction de catalyseur d'hydrogénation. Dans cette opération lors de la séparation on n'élimine pas du milieu les composés très lourds qui seront donc hydrogénés avec la fraction lourde, leur séparation intervenant lors d'une purification ultérieure de l'amino-acide/ester final.

L'autre façon de déplacer l'équilibre est d'utiliser un excès de réactif, typiquement, dans mc1 un excès d'acrylonitrile ou d'acrylate d'alkyle (de méthyle en général), et dans mc2 un excès d'alcène léger. D'un point de vue procédé, chaque métathèse croisée est conduite à son terme avec l'épuisement du catalyseur de métathèse ; l'excès d'acrylate, d'acrylonitrile, ou d'éthylène est distillé pour recyclage.

Le procédé de l'invention peut être mis en oeuvre selon un mode discontinu ou continu.

Selon le mode discontinu, par exemple mettant en oeuvre en premier un composé acrylique, le procédé de l'invention est effectué dans des réacteurs consécutifs. La première réaction de métathèse croisée mc1 est effectué dans un premier réacteur, et à l'issue de cette réaction le réacteur est purgé pour éliminer le composé acrylique résiduel (l'acrylate de Me et l'acrylonitrile sont des composés plus lourds que les oléfines, mais qui ont des point d'ébullition assez bas pour envisager une distillation sous vide partiel à 100-120 °C), et la charge est transférée dans un second réacteur, qui est mis en pression avec de l'éthylène. Après éthénolyse mc2, le réacteur ramené à la pression atmosphérique est purgé pour éliminer l'éthylène résiduel, puis la charge est transférée dans un autre réacteur. Une métathèse mc1 est alors effectuée à nouveau avec un composé acrylique, de préférence identique à celui utilisé lors la première mc1 pour ne pas compliquer les séparations ultérieures. Cette alternance de mc1 et mc2 selon l'invention peut ainsi être poursuivie autant de fois que nécessaire, de préférence jusqu'à ce que la teneur en diester ou dinitrile soit inférieure à 25 % en poids, de préférence inférieure à 10 % en poids, et de manière encore plus préférée inférieure à 5 % en poids, sur le poids total de produits dans le réacteur à l'issue de la dernière réaction de métathèse croisée selon le procédé.

Selon un mode de réalisation alternatif, le procédé de l'invention est effectué en mode continu. Dans un réacteur qui se présente sous la forme d'une colonne, la charge ester gras ou nitrile gras est alimentée en tête, le catalyseur de métathèse croisée est alimenté en continu légèrement en dessous du point d'alimentation de l'ester ou nitrile gras, et de préférence dans le quart supérieur de la colonne réactive. Le composé acrylique (acrylonitrile, acide acrylique ou acrylate selon les cas) est alimenté dans la moitié supérieure de la colonne et en dessous du point d'alimentation du catalyseur. Ce dernier, étant un composé léger, remonte la colonne alors que le nitrile ou ester gras étant un composé lourd descend la colonne. Au point d'injection et de dispersion du catalyseur, la réaction de métathèse croisée se produit, concomitamment à la réaction d'homométathèse. Les produits de ces réactions, étant des composés lourds, descendent la colonne. En tête de colonne, l'éthylène produit par les réactions de métathèse croisée et d'homométathèse et le composé acrylique, ayant des tensions de vapeur élevées, sont récupérés et dirigés vers une colonne de séparation. Le composé acrylique est réinjecté dans la colonne réactive, dans la moitié supérieure, au voisinage du point d'injection de la charge fraiche. Une partie de l'éthylène produit est purgée et une autre partie est comprimée et éventuellement injectée en pied de colonne réactive au voisinage du point d'injection de la charge fraiche d'éthylène. La hauteur de la colonne, détermine en partie la pression au point d'injection de l'éthylène. Une colonne de grande hauteur permet ainsi d'atteindre des pressions d'éthylène élevées. L'éthylène étant un composé léger, il remonte alors la colonne réactive. Dans la partie inférieure de la colonne réactive, le catalyseur d'éthénolyse est injecté, c'est-à-dire entre le point d'injection du composé acrylique et le point d'injection de l'éthylène, et de préférence dans le quart inférieur de la colonne. La réaction d'éthénolyse est une réaction rapide, qui convertit préférentiellement le diester et le dinitrile respectivement en ester et nitrile gras oméga insaturé. Ces derniers sont plus légers que le diester et le dinitrile et remontent en partie la colonne réactive pour réagir à nouveau avec le composé acrylique.

Les temps de séjour des réactifs dans la colonne sont régulés par les débits d'alimentation et de soutirage. En pied de colonne réactive, on soutire un mélange riche en nitrile ester et contenant encore du nitrile ou ester gras insaturé, mais aussi le diester ou nitrile qui n'a pas été converti. Le mélange est alors envoyé sur une unité de séparation où le nitrile ou ester gras insaturé qui n'a pas réagi est séparé et retourné en tête de colonne réactive. Le nitrile-ester produit est ultérieurement séparé du dinitrile ou diester, par exemple avant ou après une étape d'hydrogénation, dans le cas où le procédé de l'invention comprend en outre une étape d'hydrogénation du nitrile-ester(acide) mono-insaturé obtenu en α,ω-aminoester (acide).

Selon un mode de réalisation avantageux, l'hydrogénation est réalisée en présence d'un catalyseur de métathèse de l'étape précédente, ce catalyseur faisant fonction de catalyseur d'hydrogénation. Au terme d'un cycle SWING de métathèses (mc1,mc2), le milieu réactionnel contenant le ruthénium est alors directement soumis à une hydrogénation. En effet, le catalyseur de métathèse au ruthénium est dégradé à l'issue de l'étape de métathèse, mais le métal est toujours présent dans le milieu réactionnel sous une forme appropriée pour l'étape d'hydrogénation. De cette façon, le composé nitrile-ester/acide insaturé contenu dans le milieu réactionnel est hydrogéné en présence du métal du catalyseur de 1^{ère} étape dans sa fonction hydrogénation.

Typiquement, l'hydrogénation est réalisée sous pression d'hydrogène et en présence d'une base. La pression est comprise entre 5 et 100 bars, de préférence entre 20 et 30 bars. La température est comprise entre 50 et 150°C, de préférence entre 80 et 100°C. La base peut être par exemple la soude, la potasse, le tertiobutylate de potassium ou l'ammoniac. La base est généralement utilisée à une teneur de 10 à 80% en mole par rapport au substrat nitrile-ester insaturé.

La réaction d'hydrogénation peut être réalisée avec ou sans solvant. Dans le cas d'une réaction en milieu solvant, les solvants préférés utilisés pour les étapes de métathèse et d'hydrogénation sont les solvants aromatiques comme le toluène ou les xylènes ou un solvant chloré comme le dichlorométhane, le chlorobenzene, le dimethylcarbonate, des alcools, de l'acide acétique...

A l'issue de cette étape d'hydrogénation réalisée sans catalyseur spécifique d'hydrogénation, le taux de transformation de la fonction nitrile en amine primaire est particulièrement élevé même sans addition de NH₃, ainsi bien entendu que la réduction de l'insaturation oléfinique sans que la fonction carboxyle n'ait été touchée.

Le catalyseur dégradé de métathèse peut éventuellement être mis en oeuvre avec en complément un catalyseur classique d'hydrogénation pour l'étape d'hydrogénation. Parmi les métaux utilisés classiquement pour l'hydrogénation, on peut citer le nickel, le palladium, le platine, le rhodium, le ruthénium ou l'iridium. De préférence, le catalyseur dégradé de métathèse pourrait être complété par du nickel de Raney ou du palladium ou ruthénium sur charbon, ruthénium sur alumine, silice ou carbure de silicium. Ainsi, dans un mode de réalisation particulier la réaction d'hydrogénation est réalisée en présence du catalyseur dégradé de métathèse issu de la première étape complété par un catalyseur d'hydrogénation conventionnel. Il peut également être mis en oeuvre en présence d'un support solide (charbon, SiC...) afin de simplifier sa récupération.

Les amino-acides ou amino-esters saturés à longue chaîne, comportant de de 6 à 17 atomes de carbone, de préférence de 8 à 17 atomes de carbone, obtenus selon le procédé de l'invention sont avantageusement utilisés comme monomères pour la synthèse de polymères, en particulier de polyamides. La synthèse de ces polymères est facilitée par l'absence d'impuretés (co-produits d'homométathèse) des monomères fabriqués selon le procédé de l'invention.

L'invention a encore pour objet des polymères obtenus par polymérisation des α,ω-aminoesters (acides) synthétisés selon les procédés définis ci-dessus.

### EXEMPLES

Les exemples suivants illustrent l'invention sans la limiter.

### Exemple comparatif (non conforme à l'invention)

Dans un réacteur en verre double-enveloppe de 250 ml, muni d'une agitation mécanique, d'un réfrigérant, d'une sonde de température et d'une arrivée d'azote, on charge 5g de 10-undécènenitrile (30 mmol), 5,2g d'acrylate de méthyle (60 mmol) et 50g de toluène. On purge à l'azote et on chauffe à 100°C. On ajoute dans le réacteur sur une période de 3 heures via une seringue et un pousse-seringue 0,55 mg de catalyseur Hoveyda-Grubbs de seconde génération (Aldrich, 0,003% mol par rapport au 10-undécènenitrile) dissous dans 5g de toluène. La conversion du 10-undécènitrile déterminée par CPG est de 69%. La sélectivité en nitrile-ester C12 insaturé (produit de métathèse croisée) est de 19%.

### Exemple 1 conforme à l'invention

Le 10-undecenenitrile est purifié par passage sur alumine. Le toluène est purifié par passage sur tamis moléculaire.

Dans un réacteur en verre double-enveloppe de 250 ml, muni d'une agitation mécanique, d'un réfrigérant, d'une sonde de température et d'une arrivée d'azote, on charge 5g de 10-undécènenitrile (30 mmol), 5,2g d'acrylate de méthyle (60 mmol) et 50g de toluène. On purge à l'azote et on chauffe à 100°C. On ajoute dans le réacteur sur une période de 3 heures, via une seringue et un pousse-seringue, 0,38 mg de catalyseur Hoveyda-Grubbs de seconde génération (Aldrich, 0,002% mol par rapport au 10-undécènenitrile) dissous dans 5g de toluène. L'éthylène produit est éliminé en continu.

A la fin de l'ajout du catalyseur, après une purge de l'acrylate de méthyle résiduel, le mélange réactionnel est transféré dans un autoclave de 300ml. On ajoute 12mg de catalyseur de Grubbs de 1 ère génération (0,05% mol par rapport au 10-undécènenitrile). On purge à l'azote et on pressurise sous 5 bars d'éthylène. On chauffe à 50°C et on laisse réagir 30 minutes.

Après refroidissement et décompression, puis purge à l'azote, le mélange réactionnel est réintroduit dans le montage à pression atmosphérique. On ajoute 2,6g d'acrylate de méthyle (30 mmol). On purge à l'azote et on chauffe à 100°C, puis on ajoute, sur une période de 1 heure, 0,19 mg de catalyseur Hoveyda-Grubbs de seconde génération (0,001% mol par rapport au 10-undécènenitrile) dissous dans 5g de toluène. La conversion du 10-undécènitrile déterminée par CPG est de 90%. La sélectivité en nitrile-ester C12 insaturé (produit de métathèse croisée) est de 76%.

Ces exemples montrent que pour une même teneur en catalyseur Hoveyda-Grubbs de seconde génération, on obtient avec le procédé selon l'invention (Exemple 1) une meilleure conversion du 10-undécènenitrile et une sélectivité beaucoup plus élevée en produit de métathèse croisée.

### Etape d'hydrogénation

Le mélange réactionnel obtenu dans l'exemple 1 est transféré dans un autoclave de 300ml. On ajoute 0,5g de Nickel de Raney préalablement lavé au méthanol. On purge l'autoclave à l'azote, puis on introduit 5 bars d'ammoniac et 30 bars d'hydrogène. On chauffe à 100°C et on laisse réagir 4 heures.

La conversion en nitrile-ester C12 insaturé déterminée par chromatographie en phase gaz est supérieure à 99%. La sélectivité en amino-ester C12 saturé est supérieure à 95%.

### Etape de polymérisation

Le produit issu de l'hydrogénation est récupéré par distillation sous pression réduite et est transféré dans un autoclave. L'autoclave est purgé à l'azote, puis placé sous une pression réduite de 50 mbars. On chauffe à 210°C et on laisse réagir 2 heures. On obtient ainsi le polyamide 12.

## Revendications

1. Procédé de synthèse d'un nitrile-ester(acide) mono-insaturé par métathèses croisées à partir d'un composé I insaturé comportant au moins une fonction trivalente, nitrile, acide ou ester, **caractérisé en ce qu'**il comprend une étape dans laquelle on alterne de manière consécutive deux types de métathèse croisée :
- un premier type de métathèse croisée mc1 avec un composé acrylique II choisi parmi l'acrylonitrile, l'acide acrylique ou un ester acrylique, l'un des composés I ou II comportant une fonction nitrile et l'autre de ces composés II ou I une fonction acide ou ester, et
- un deuxième type de métathèse croisée mc2 avec un composé alcène léger III en C2 à C4, de préférence avec l'éthylène.

2. Procédé selon la revendication 1, dans lequel le composé I insaturé est de formule :
R1-CH=CH-[(CH₂)_{q}-CH=CH]ₚ -(CH₂)ₙ-R2 dans laquelle :
R1 est H, un radical alkyle de 1 à 11 atomes de carbone comportant le cas échéant une fonction hydroxyle, ou (CH₂)ₘ-R4
m est un indice entier compris dans la gamme de 0 à 11,
n est un indice entier compris dans la gamme de 2 à 13,
p est un indice entier égal à 0, 1 ou 2,
q est un indice égal à 0 ou 1,
R2 est COOR5 ou CN,
R4 est H ou R2
R5 est choisi parmi : H, un radical alkyle de 1 à 11 atomes de carbone, un radical comportant deux ou trois atomes de carbone porteur de une ou deux fonction(s) hydroxyle(s), ou encore le reste d'un di-glycéride ou d'un tri-glycéride dans lequel chaque acide gras dudit reste de glycéride est indifféremment saturé ou insaturé.

3. Procédé selon la revendication 2, dans lequel l'indice p est égal à 0, R1 est égal à H, et le cycle de métathèses commence de préférence par une métathèse mc1 sur le composé I de formule :
CH₂=CH-(CH₂)ₙ-R2

4. Procédé selon la revendication 2 ou 3, dans lequel le composé I insaturé est un acide ou ester gras de formule :
CH₂=CH-(CH₂)ₙ-COOR5 dans laquelle
R5 est H ou un radical alkyle de 1 à 11 atomes de carbone

5. Procédé selon la revendication 2 ou 3, dans lequel le composé I insaturé est un nitrile de formule :
CH₂=CH-(CH₂)ₙ-CN

6. Procédé selon la revendication 2, dans lequel l'indice p est différent de 0 et/ou R1 est différent de H, et le cycle de métathèses commence de préférence par une métathèse mc2.

7. Procédé selon la revendication 4, **caractérisé en ce que** la réaction de métathèse mc1 est réalisée entre l'acrylonitrile et un composé I choisi parmi l'acide 9-décénoïque, le 9-décénoate de méthyle, l'acide 10-undécénoïque, le 10-undécénoate de méthyle, l'acide oléique, l'oléate de méthyle, l'acide 9-octadécènedioïque, le 9-octadécènedioate de méthyle, l'acide érucique, l'érucate de méthyle, l'acide 12-tridécénoïque et le 12-tridécénoate de méthyle.

8. Procédé selon la revendication 5, **caractérisé en ce que** la réaction de métathèse mc1 est réalisée entre une ester(acide) acrylique et un composé I choisi parmi le 9-décènenitrile, le 10-undécènenitrile, le 9-octadécènenitrile ou oléonitrile, le 9-octadécènedinitrile, l'éruconitrile, le 12-tridécénonitrile.

9. Procédé selon la revendication 6, **caractérisé en ce que** la réaction de métathèse croisée mc2 est réalisée entre un alcène léger III en C2 à C4 et un composé I choisi parmi les huiles naturelles, les triglycérides, les esters et acides gras polyinsaturés, et leurs mélanges.

10. Procédé selon l'une quelconque des revendications précédentes, **caractérisé en ce qu'**il comprend au moins la succession d'étapes mc1-mc2-mc1 ou mc2-mc1-mc2.

11. Procédé selon l'une quelconque des revendications 1 ou 2, **caractérisé en ce que** le composé I est un triglycéride et le procédé comprend au moins la succession d'étapes mc2-mc1, éventuellement suivie d'une étape d'alcoolyse ou d'hydrolyse.

12. Procédé selon l'une des revendications 1 à 11, **caractérisé en ce que** la métathèse croisée est réalisée en présence d'un catalyseur au ruthénium choisi parmi les catalyseurs chargés ou non-chargés de formule générale :
(X1)ₐ (X2)_{b}Ru(carbène C) (L1)_{c}(L2)_{d},(L3)ₑ,
dans laquelle :
• a, b, c, d et e sont des nombres entiers, identiques ou différents, avec a et b égaux à 0, 1 ou 2 ; c, d et e égaux à 0, 1, 2, 3 ou 4;
• X1 et X2, identiques ou différents, représentent chacun un ligand mono-ou multi-chélatant, chargé ou non, de préférence choisi parmi les halogénures, le sulfate, le carbonate, les carboxylates, les alcoolates, les phénolates, les amidures, le tosylate, l'hexafluorophosphate, le tétrafluoroborate, le bis-triflylamidure, le tétraphénylborate et dérivés. X1 ou X2 peuvent être liés à Y1 ou Y2 (L1 ou L2) ou au (carbène C) de façon à former un ligand bidenté ou chélate sur le ruthénium ; et
• L1, L2, et L3 identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéthers, ou un carbène hétérocyclique
L1, L2 ou L3 pouvant être liés au (carbène C) de façon à former un ligand bidenté ou chélate, ou tridenté, le (carbène C) étant représenté par la formule générale : C_(R1)_(R2) pour laquelle R1 et R2 sont des groupes identiques ou différents, tels que l'hydrogène ou tout autre groupe hydrocarboné, fonctionnalisé ou non, de type saturé, insaturé, cyclique, aromatique, branché et/ou linéaire.

13. Procédé selon la revendication 12, **caractérisé en ce que** la quantité de catalyseur présente pendant la réaction de métathèse est **caractérisée par** un turnover number, égal au rapport molaire [réactif qui a réagi] / [catalyseur], compris dans la gamme de 5 000 à 200 000, de préférence 10 000 à 50 000.

14. Procédé selon l'une des revendications 12 ou 13, **caractérisé en ce que** la réaction de métathèse mc1 est réalisée en présence d'un catalyseur dans lequel L1, L2 et/ou L3 est un carbène hétérocyclique.

15. Procédé selon l'une quelconque des revendications 12 à 14, **caractérisé en ce que** la réaction de métathèse mc2 est réalisée en présence d'un catalyseur dans lequel L1, L2, et L3 ne sont pas des carbènes hétérocycliques.

16. Procédé selon l'une quelconque des revendications 12 à 15, **caractérisé en ce que** la métathèse croisée mc1 et/ou mc2 est réalisée en présence d'un catalyseur au ruthénium, répondant à l'une des formules ci-dessous :

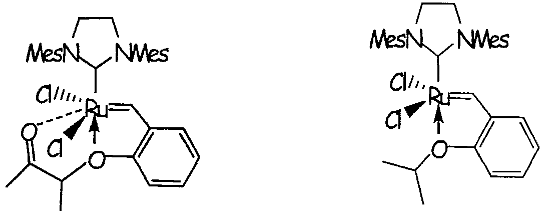

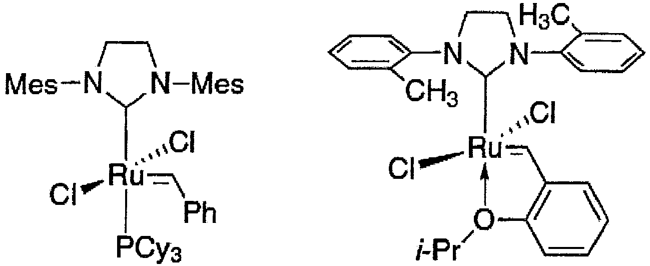

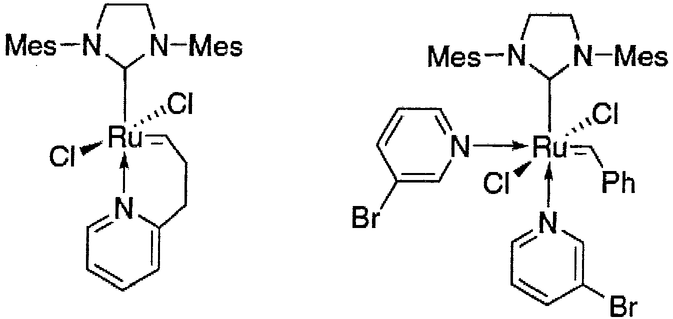

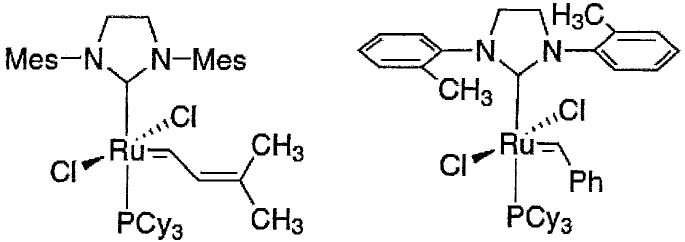

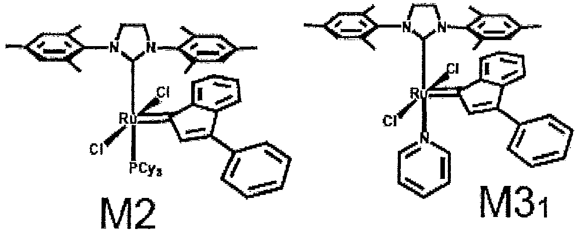

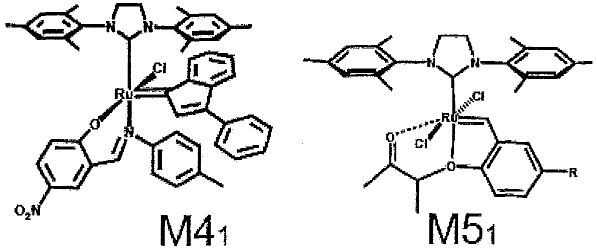

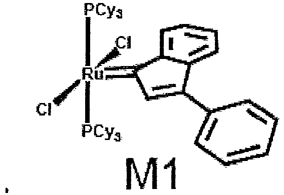

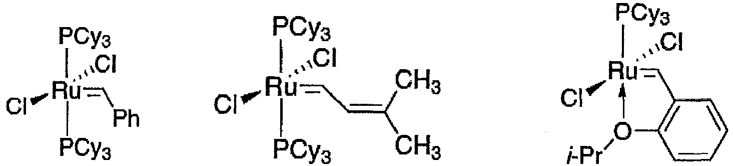

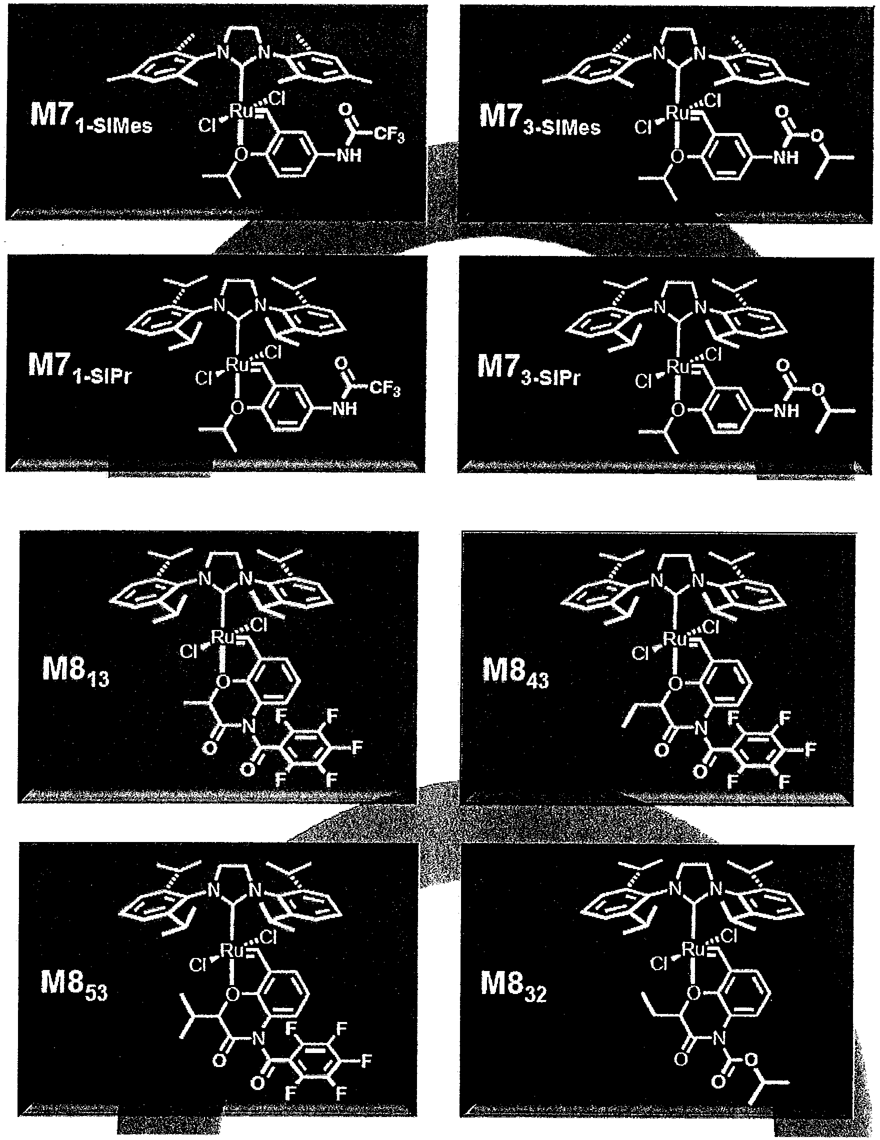

17. Procédé selon l'une quelconque des revendications 1 à 16, comprenant en outre une étape d'hydrogénation du nitrile-ester(acide) mono-insaturé obtenu, de sorte que l'on obtient un α,ω-aminoester (acide) saturé.

18. Procédé selon la revendication 17, **caractérisé en ce que** l'hydrogénation est réalisée en présence d'un catalyseur de métathèse de l'étape précédente, ce catalyseur faisant fonction de catalyseur d'hydrogénation.

19. Procédé selon l'une quelconque des revendications 17 ou 18 comprenant en outre une étape de synthèse de polyamide par polymérisation utilisant l'α,ω-aminoester(acide) obtenu à l'issue de l'étape d'hydrogénation.

20. Polymère obtenu par polymérisation d'α,ω-aminoester (acide) synthétisé selon le procédé de l'une quelconque des revendications 17 ou 18.

## Patentansprüche

1. Verfahren zur Synthese von einem (einer) monoungesättigten Nitrilester (säure) durch Kreuzmetathesen aus einer ungesättigten Verbindung I, die mindestens eine trivalente, Nitril-, Säure- oder Ester-Funktion umfasst,
**dadurch gekennzeichnet, dass** dieses einen Schritt umfasst, in dem konsekutiv zwei Typen einer Kreuzmetathese alternierend eingesetzt werden:
- ein erster Typ einer Kreuzmetathese mc1 mit einer Acryl-Verbindung II, die ausgewählt wird aus Acrylnitril, Acrylsäure oder einem Acrylester, wobei eine der Verbindungen I oder II eine Nitril-Funktion und die andere dieser Verbindungen II oder I eine Säure- oder Ester-Funktion umfasst, und
- ein zweiter Typ einer Kreuzmetathese mc2 mit einer leichten C2-C4-Alken-Verbindung III, vorzugsweise mit Ethylen.

2. Verfahren nach Anspruch 1, wobei die ungesättigte Verbindung I die Formel aufweist:
R1-CH=CH-[(CH₂)_{q}-CH=CH]ₚ-(CH₂)ₙ-R2,
wobei:
R1 H, ein Alkyl-Rest mit 1 bis 11 Kohlenstoffatomen, gegebenenfalls umfassend eine Hydroxyl-Funktion, oder (CH₂)ₘ-R4 ist,
m ein ganzzahliger Index in dem Bereich von 0 bis 11 ist,
n ein ganzzahliger Index in dem Bereich von 2 bis 13 ist,
p ein ganzzahliger Index gleich 0, 1 oder 2 ist,
q ein Index gleich 0 oder 1 ist,
R2 COOR5 oder CN ist,
R4 H oder R2 ist,
R5 ausgewählt wird aus: H, einem Alkyl-Rest mit 1 bis 11 Kohlenstoffatomen, einem Rest umfassend zwei oder drei Kohlenstoffatome, der eine oder zwei Hydroxyl-Funktion (en) trägt, oder auch einem Diglycerid- oder Triglycerid-Rest, wobei jede Fettsäure des Glycerid-Rests entweder gesättigt oder ungesättigt ist.

3. Verfahren nach Anspruch 2, wobei der Index p gleich 0 ist, R1 gleich H ist, und der Metathesenzyklus vorzugsweise durch eine Metathese mc1 an der Verbindung I der folgenden Formel beginnt:
CH₂=CH- (CH₂)ₙ-R2

4. Verfahren nach Anspruch 2 oder 3, wobei die ungesättigte Verbindung I eine Fettsäure oder ein Fettsäureester der folgenden Formel ist:
CH₂=CH-(CH₂)ₙ-COOR5,
wobei:
R5 H oder ein Alkyl-Rest mit 1 bis 11 Kohlenstoffatomen ist.

5. Verfahren nach Anspruch 2 oder 3, wobei die ungesättigte Verbindung I ein Nitril der folgenden Formel ist:
CH₂=CH- (CH₂)ₙ-CN

6. Verfahren nach Anspruch 2, wobei der Index p von 0 verschieden ist, und/oder R1 von H verschieden ist, und der Metathesenzyklus vorzugsweise durch eine Metathese mc2 beginnt.

7. Verfahren nach Anspruch 4, **dadurch gekennzeichnet, dass** die Reaktion der Metathese mc1 zwischen Acrylnitril und einer Verbindung I durchgeführt wird, die ausgewählt wird aus 9-Decensäure, Methyl-9-decenoat, 10-Undecensäure, Methyl-10-undecenoat, Oleinsäure, Methyloleat, 9-Octadecendionsäure, Methyl-9-octadecendioat, Erucasäure, Methylerucat, 12-Tridecensäure und Methyl-12-tridecenoat.

8. Verfahren nach Anspruch 5, **dadurch gekennzeichnet, dass** die Reaktion der Metathese mc1 zwischen einem (einer) Acrylester(säure) und einer Verbindung I durchgeführt wird, die ausgewählt wird aus 9-Decennitril, 10-Undecennitril, 9-Octadecennitril oder Oleonitril, 9-Octadecendinitril, Eruconitril und 12-Tridecennitril.

9. Verfahren nach Anspruch 6, **dadurch gekennzeichnet, dass** die Reaktion der Kreuzmetathese mc2 zwischen einem leichten C2-C4-Alken III und einer Verbindung I durchgeführt wird, die ausgewählt wird aus natürlichen Ölen, Triglyceriden, polyungesättigten Fettsäureestern und -säuren und ihren Mischungen.

10. Verfahren nach einem der vorhergehenden Ansprüche, **dadurch gekennzeichnet, dass** dieses mindestens die aufeinanderfolgenden Schritte mc1-mc2-mc1 oder mc2-mc1-mc2 umfasst.

11. Verfahren nach einem der Ansprüche 1 oder 2, **dadurch gekennzeichnet, dass** die Verbindung I ein Triglycerid ist, und das Verfahren mindestens die aufeinanderfolgenden Schritte mc2-mc1, gegebenenfalls gefolgt von einem Alkoholyse- oder Hydrolyse-Schritt, umfasst.

12. Verfahren nach einem der Ansprüche 1 bis 11, **dadurch gekennzeichnet, dass** die Kreuzmetathese in Anwesenheit eines Ruthenium-Katalysators durchgeführt wird, der ausgewählt wird aus geladenen oder ungeladenen Katalysatoren der allgemeinen Formel:
(X1)ₐ(X2)_{b}Ru(Carben C) (L1)_{c}(L2)_{d}(L3)ₑ,
wobei:
- a, b, c, d und e gleiche oder verschiedene ganze Zahlen sind, wobei a und b gleich 0, 1 oder 2 sind; c, d und e gleich 0, 1, 2, 3 oder 4 sind;
- X1 und X2, die gleich oder verschieden sind, jeweils einen mono- oder multi-chelatbildenden, geladenen oder ungeladenen Liganden darstellen, der vorzugsweise ausgewählt wird aus Halogeniden, Sulfat, Carbonat, Carboxylaten, Alkoholaten, Phenolaten, Amiden, Tosylat, Hexafluorphosphat, Tetrafluorborat, Bis-Triflylamid, Tetraphenylborat und Derivaten, wobei X1 oder X2 mit Y1 oder Y2 (L1 oder L2) oder mit (Carben C) verbunden sein können, um einen zweizähnigen oder Chelat-Liganden an Ruthenium zu bilden; und
- L1, L2 und L3, die gleich oder verschieden sind, Elektronendonator-Liganden sind, wie Phosphin, Phosphit, Phosphonit, Phosphinit, Arsin, Stilbin, ein Olefin oder ein Aromat, eine Carbonyl-Verbindung, ein Ether, ein Alkohol, ein Amin, ein Pyridin oder Derivat, ein Imin, ein Thioether oder ein heterozyklisches Carben,
wobei L1, L2 oder L3 mit (Carben C) verbunden sein können, um einen zweizähnigen oder Chelat- oder dreizähnigen Liganden zu bilden, wobei (Carben C) dargestellt wird durch die allgemeine Formel: C_(R1)_(R2), wobei R1 und R2 gleiche oder verschiedene Gruppen sind, wie Wasserstoff oder jede andere Kohlenwasserstoff-Gruppe, funktionalisiert oder nicht, vom gesättigten, ungesättigten, zyklischen, aromatischen, verzweigten und/oder linearen Typ.

13. Verfahren nach Anspruch 12, **dadurch gekennzeichnet, dass** die Menge an Katalysator, die während der Reaktion der Metathese vorhanden ist, gekennzeichnet wird durch eine Umsatzzahl gleich einem Molekularverhältnis [Reagenz, das reagiert hat] / [Katalysator] in dem Bereich von 5.000 bis 200.000, vorzugsweise 10.000 bis 50.000.

14. Verfahren nach einem der Ansprüche 12 oder 13, **dadurch gekennzeichnet, dass** die Reaktion der Metathese mc1 in Anwesenheit eines Katalysators durchgeführt wird, wobei L1, L2 und/oder L3 ein heterozyklisches Carben ist.

15. Verfahren nach einem der Ansprüche 12 bis 14, **dadurch gekennzeichnet, dass** die Reaktion der Metathese mc2 in Anwesenheit eines Katalysators durchgeführt wird, wobei L1, L2 und L3 keine heterozyklischen Carbene sind.

16. Verfahren nach einem der Ansprüche 12 bis 15, **dadurch gekennzeichnet, dass** die Kreuzmetathese mc1 und/oder mc2 in Anwesenheit eines Ruthenium-Katalysators durchgeführt wird, der eine der folgenden Formeln aufweist:

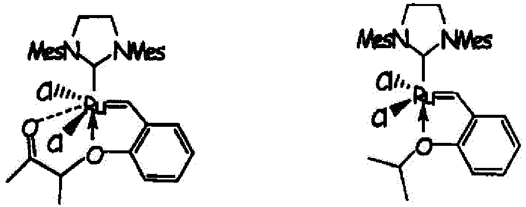

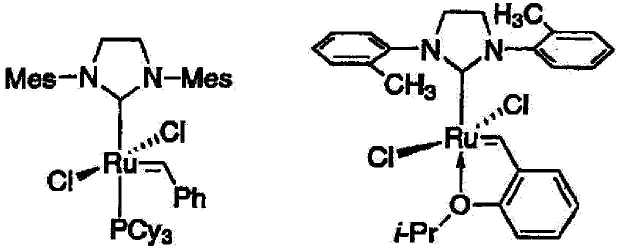

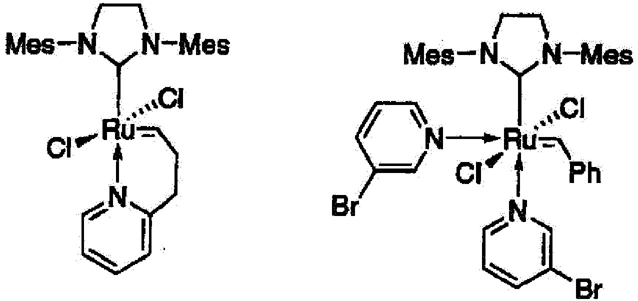

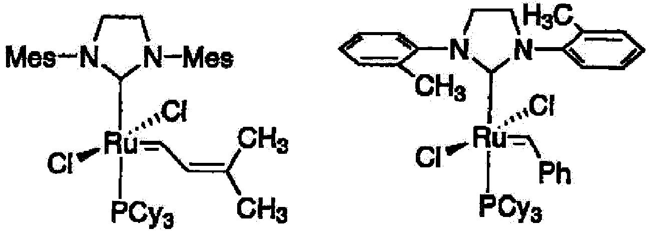

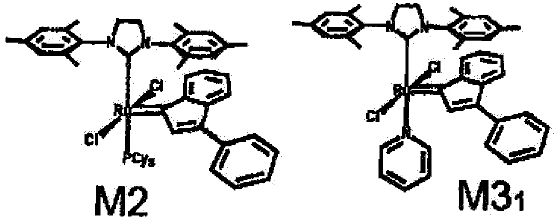

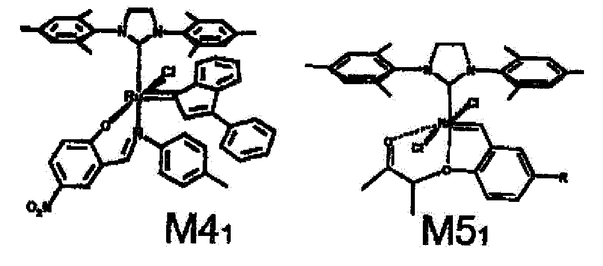

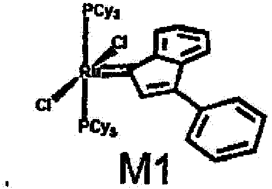

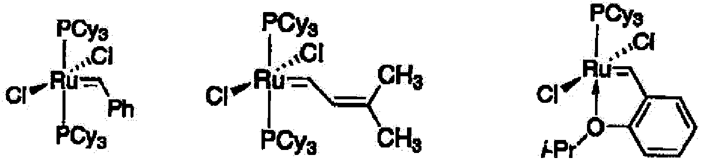

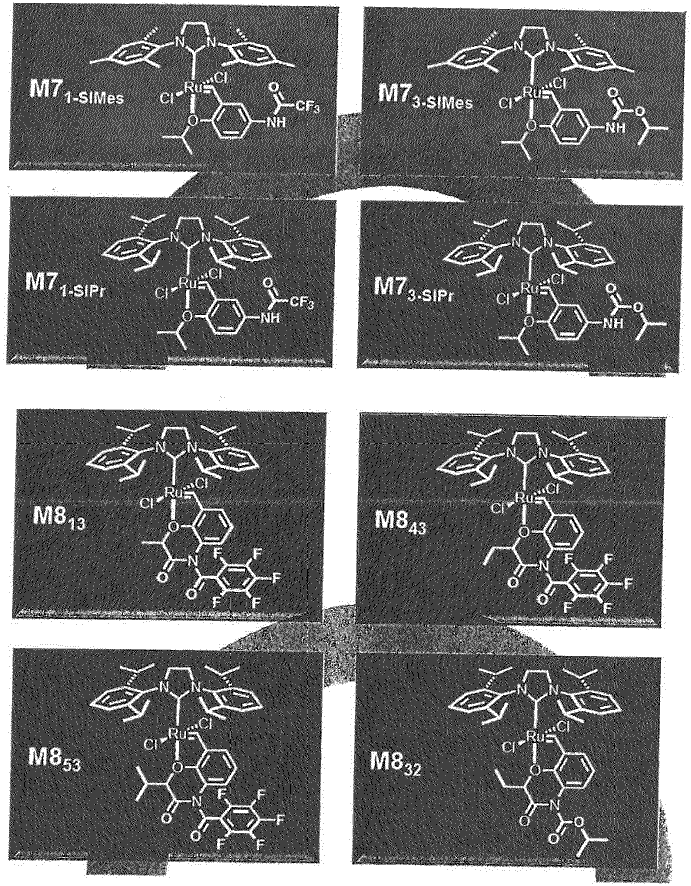

17. Verfahren nach einem der Ansprüche 1 bis 16, ferner umfassend einen Schritt der Hydrierung des (der) erhaltenen mono-ungesättigten Nitrilesters (säure), um gesättigten (gesättigte) α,ω-Aminoester (säure) zu erhalten.

18. Verfahren nach Anspruch 17, **dadurch gekennzeichnet, dass** die Hydrierung in Anwesenheit eines Katalysators der Metathese des vorhergehenden Schritts durchgeführt wird, wobei dieser Katalysator als Hydrierungskatalysator dient.

19. Verfahren nach einem der Ansprüche 17 oder 18, ferner umfassend einen Schritt der Synthese von Polyamid durch Polymerisation unter Verwendung des (der) α,ω-Aminoesters (säure), der (die) nach dem Hydrierungsschritt erhalten wird.

20. Polymer, welches durch Polymerisation eines (einer) α,ω-Aminoesters (säure) erhalten wird, der (die) gemäß dem Verfahren nach einem der Ansprüche 17 oder 18 synthetisiert wird.

## Claims

1. A process for the synthesis of a monounsaturated nitrile-ester (acid) by cross metathesis starting from an unsaturated compound I comprising at least one trivalent nitrile, acid or ester functional group, **characterized in that** it comprises a stage in which two types of cross metathesis are alternated consecutively:
- a first type of cross metathesis mc1 with an acrylic compound II chosen from acrylonitrile, acrylic acid or an acrylic ester, one of the compounds I or II comprising a nitrile functional group and the other of these compounds II or I comprising an acid or ester functional group, and
- a second type of cross metathesis mc2 with a light C₂ to C₄ alkene compound III, preferably with ethylene.

2. The process as claimed in claim 1, in which unsaturated compound I is of formula:
R₁-CH=CH-[(CH₂)_{q}-CH=CH]ₚ -(CH₂)ₙ-R2 in which:
R₁ is H, an alkyl radical having from 1 to 11 carbon atoms, if appropriate comprising a hydroxyl functional group, or a (CH₂)ₘ-R₄ radical,
m is a whole index within the range from 0 to 11,
n is a whole index within the range from 2 to 13,
p is a whole index equal to 0, 1 or 2,
q is an index equal to 0 or 1,
R₂ is COOR₅ or CN,
R₄ is H or R₂,
R₅ is chosen from: H, an alkyl radical having from 1 to 11 carbon atoms, a radical comprising two or three carbon atoms carrying one or two hydroxyl functional group(s), or also the residue of a diglyceride or of a triglyceride in which each fatty acid of said glyceride residue is without distinction saturated or unsaturated.

3. The process as claimed in claim 2, in which the index p is equal to 0, R₁ is equal to H and the cycle of metatheses preferably begins with a mc1 metathesis on the compound I of formula:
CH₂=CH-(CH₂)ₙ-R₂.

4. The process as claimed in claim 2 or 3, in which the unsaturated compound I is a fatty acid or ester of formula:
CH₂=CH-(CH₂)ₙ-COOR₅, in which
R₅ is H or an alkyl radical having from 1 to 11 carbon atoms.

5. The process as claimed in claim 2 or 3, in which the unsaturated compound **I** is a nitrile of formula:
CH₂=CH-(CH₂)ₙ-CN.

6. The process as claimed in claim 2, in which the index p is other than 0 and/or R₁ is other than H, and the cycle of metatheses preferably begins with a mc2 metathesis.

7. The process as claimed in claim 4, **characterized in that** the mc1 metathesis reaction is carried out with acrylonitrile and a compound **I** chosen from 9-decenoic acid, methyl 9-decenoate, 10-undecenoic acid, methyl 10-undecenoate, oleic acid, methyl oleate, 9-octadecenedioic acid, methyl 9-octadecenedioate, erucic acid, methyl erucate, 12-tridecenoic acid and methyl 12-tridecenoate.

8. The process as claimed in claim 5, **characterized in that** the mc1 metathesis reaction is carried out between an acrylic ester (acid) and a compound **I** chosen from 9-decenenitrile, 10-undecenenitrile, 9-octadecenenitrile or oleonitrile, 9-octadecenedinitrile, eruconitrile or 12-tridecenonitrile.

9. The process as claimed in claim 6, **characterized in that** the mc2 cross metathesis reaction is carried out between a light C₂ to C₄ alkene **III** and a compound **I** chosen from natural oils, triglycerides, polyunsaturated fatty esters and acids, and their mixtures.

10. The process as claimed in any one of the preceding claims, **characterized in that** it comprises at least the sequence of stages mc1-mc2-mc1 or mc2-mc1-mc2.

11. The process as claimed in either one of claims 1 and 2, **characterized in that** the compound I is a triglyceride and the process comprises at least the sequence of stages mc2-mc1, optionally followed by an alcoholysis or hydrolysis stage.

12. The process as claimed in one of claims 1 to 11, **characterized in that** the cross metathesis is carried out in the presence of a ruthenium catalyst chosen from charged or uncharged catalysts of general formula:
(X1)ₐ(X2)_{b}Ru(carbene C)(L1)_{c}(L2)_{d}(L3)ₑ,
in which:
• a, b, c, d and e are identical or different integers, with a and b equal to 0, 1 or 2; c, d and e equal to 0, 1, 2, 3 or 4;
• X1 and X2, which are identical or different, each represent a charged or uncharged and monochelating or polychelating ligand, preferably chosen from halides, sulfate, carbonate, carboxylates, alkoxides, phenolates, amides, tosylate, hexafluorophosphate, tetrafluoroborate, bis(triflyl)amide, tetraphenylborate and derivatives. X1 or X2 can be bonded to Y1 or Y2 (L1 or L2) or to the (carbene C) so as to form a bidentate or chelate ligand on the ruthenium; and
• L1, L2 and L3, which are identical or different, are electron-donating ligands, such as phosphine, phosphite, phosphonite, phosphinite, arsine, stilbene, an olefin or an aromatic compound, a carbonyl compound, an ether, an alcohol, an amine, a pyridine or derivative, an imine, a thioether or a heterocyclic carbene;
it being possible for L1, L2 or L3 to be bonded to the (carbene C) so as to form a bidentate or chelate ligand, or a tridentate ligand, the (carbene C) being represented by the general formula: C_(R1)_(R2), for which R₁ and R₂ are identical or different groups, such as hydrogen or any other functionalized or nonfunctionalized hydrocarbon group of saturated, unsaturated, cyclic, aromatic, branched and/or linear type.

13. The process as claimed in claim 12, **characterized in that** the amount of catalyst present during the metathesis reaction is **characterized by** a turnover number, equal to the [reactant which has reacted]/[catalyst] molar ratio, within the range from 5000 to 200 000 and preferably from 10 000 to 50 000.

14. The process as claimed in either of claims 12 and 13, **characterized in that** the mc1 metathesis reaction is carried out in the presence of a catalyst in which L1, L2 and/or L3 is a heterocyclic carbene.

15. The process as claimed in any one of claims 12 to 14, **characterized in that** the mc2 metathesis reaction is carried out in the presence of a catalyst in which L1, L2 and L3 are not heterocyclic carbenes.

16. The process as claimed in any one of claims 12 to 15, **characterized in that** the mc1 and/or mc2 cross metathesis is carried out in the presence of a ruthenium catalyst corresponding to one of the following formulae:

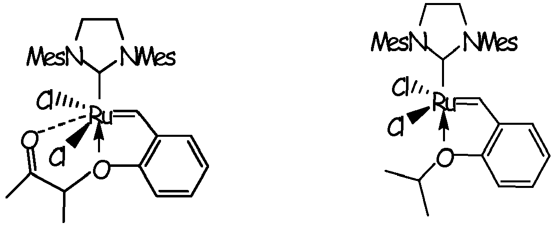

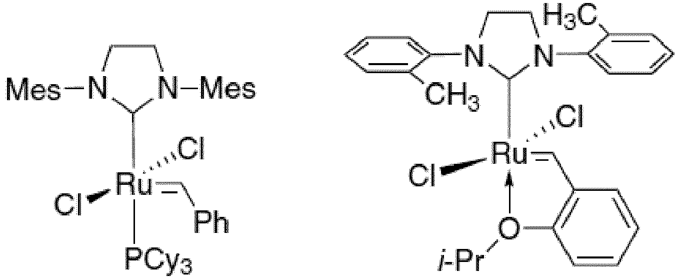

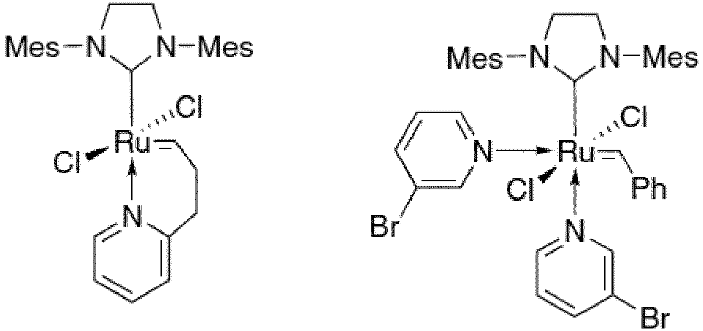

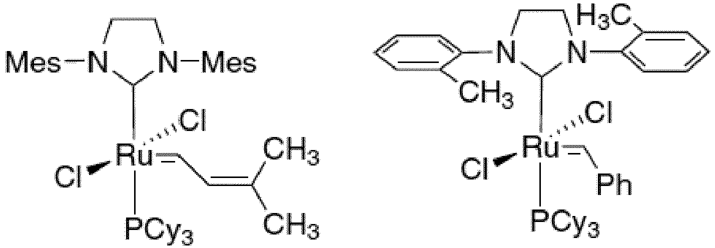

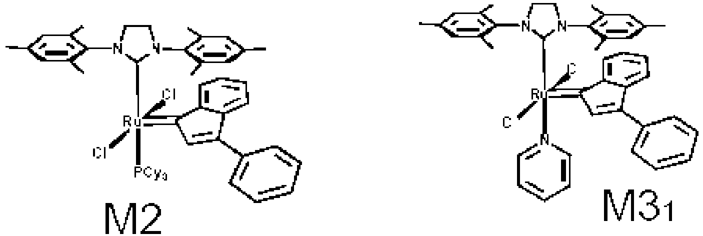

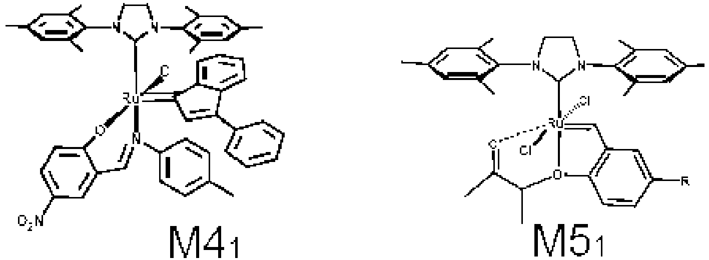

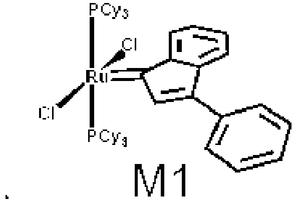

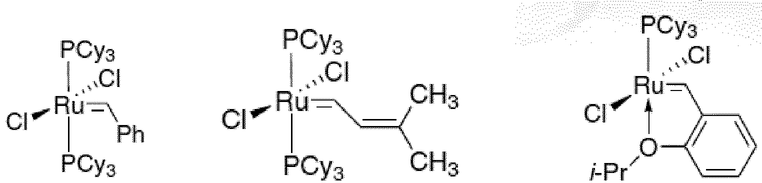

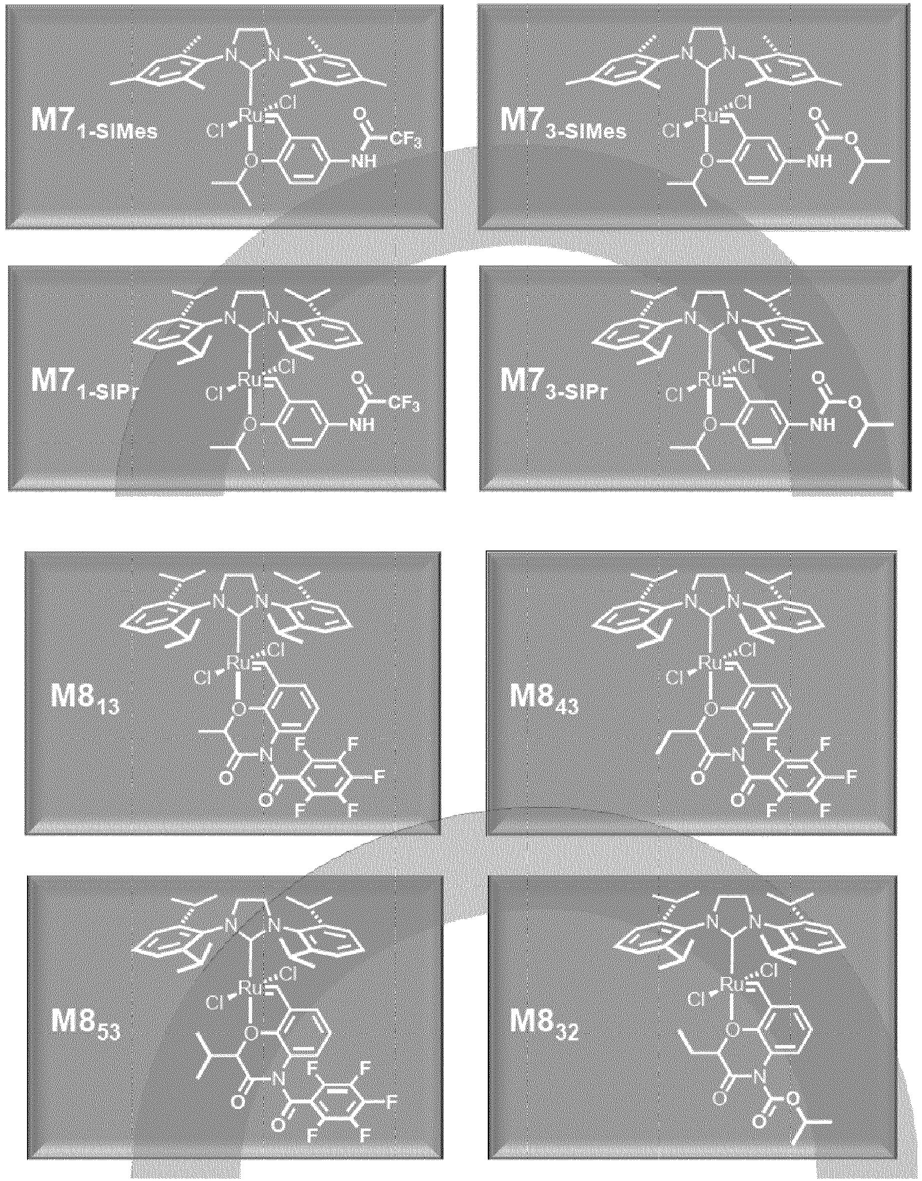

17. The process as claimed in any of claims 1 to 16, additionally comprising a stage of hydrogenation of the monounsaturated nitrile-ester (acid) obtained, so that a saturated α,ω-amino ester (acid) is obtained.

18. The process as claimed in claim 17, **characterized in that** the hydrogenation is carried out in the presence of a metathesis catalyst from the preceding stage, this catalyst operating as hydrogenation catalyst.

19. The process as claimed in either one of claims 17 and 18, additionally comprising a stage of synthesis of polyamide by polymerization using the α,ω-amino ester (acid) obtained on conclusion of the hydrogenation stage.

20. A polymer, obtained by polymerization of α,ω-amino ester (acid) synthesized according to the process of either one of claims 17 and 18.
